(19) Europäisches Patentamt
European Patent Office
Office européen des brevets

(11) **EP 4 647 438 A1**

(12) **EUROPEAN PATENT APPLICATION**

(43) Date of publication:
**12.11.2025 Bulletin 2025/46**

(21) Application number: **24174890.4**

(22) Date of filing: **08.05.2024**

(51) International Patent Classification (IPC):
**C07K 14/005** (2006.01)    **C12N 7/00** (2006.01)

(52) Cooperative Patent Classification (CPC):
**C07K 14/005; C12N 7/00;** A61K 38/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **A-Spectar GmbH**
**2100 Korneuburg (AT)**

(72) Inventors:
• **SCHÖNFELD, Wolfgang**
**2100 Korneuburg (AT)**

• **DU PLESSIS, Lana**
**6570 Knysna (ZA)**

(74) Representative: **SONN Patentanwälte GmbH & Co KG**
**Riemergasse 14**
**1010 Wien (AT)**

Remarks:
The complete document including Reference Table(s) and the Sequence Listing(s) can be downloaded from the EPO website

(54) **POLYPEPTIDE COMPRISING A CAPSID PROTEIN SEQUENCE**

(57)    The present invention discloses a polypeptide comprising a capsid protein sequence consisting of eight core regions and seven loop modification regions,
wherein the eight core regions are defined, respectively, by amino acid positions M1 to S309, P319 to R328, T340 to T342, M349 to F352, W359 to F362, V368 to Q373, T383 to T387 and T392 to N644 in the amino acid sequence according to SEQ ID NO: 1,
wherein the eight core regions of the capsid protein sequence have at least 70 % sequence identity to the eight core regions of the amino acid sequence according to SEQ ID NO: 1 or SEQ ID NO: 2 at least in the range from R32 to N570,
wherein the seven loop modification regions are defined, respectively, by amino acid positions A310 to I318, H329 to I339, I343 to T348, D353 to E358, A363 to D367, T374 to A382, and A388 to V391 in the amino acid sequence according to SEQ ID NO: 1,
wherein at least one loop modification region of the capsid protein sequence differs from the corresponding loop modification region of the amino acid sequences according to SEQ ID NO: 1 and SEQ ID NO: 2 at least in that the at least one loop modification region comprises
- a peptide insert sequence comprising at least 3 amino acids, so that the at least one loop modification region of the capsid protein sequence is extended with respect to the corresponding loop modification region of the amino acid sequences according to SEQ ID NO: 1 and SEQ ID NO: 2 by said number of amino acids; and/or
- a cysteine residue,

and Virus-like Nanoparticles (VLNPs) comprising such polypeptides.

EP 4 647 438 A1

**Description**

The invention relates to polypeptides comprising a capsid protein sequence and Virus-like Nanoparticles (VLNPs) comprising such polypeptides.

The delivery of pharmaceuticals and biologicals *in vivo* is often the biggest stumbling block to achieve the desired efficacy of delivery of these drugs. Generally, most drugs cannot be delivered to a specific diseased cell or cellular receptor by traditional methods whether by inhalation, ingestion or injection. Moreover, drugs may be rapidly degraded or metabolised, and often characterized by overall low bioavailability. Non-targeted administration may cause undesired systemic side-effects.

In this respect, chemotherapeutic drugs for treatment of various cancers are some of the most toxic and harmful drugs. They are generally selected for anti-tumor activity, but they cannot specifically and selectively target tumor cells alone with no off target uptake in normal cells. Traditional chemotherapy treatment produces unfavorable side-effects, i.e. hair loss, nail loss, mouth ulcers, diarrhea, nausea and vomiting, fatigue and organ toxicities.

Specific targeting of drugs to a selected cell for some therapeutic advantage would be highly advantageous. Drug delivery systems that allow drug targeting to a specific cell or site allow for the administration of an increased drug concentration to be achieved at the specific site, regardless of route of administration of the drug. Therefore, targeted drug delivery will reduce off target uptake of toxic drugs, thus leading to less adverse reactions with more rapid recovery rates. A further benefit of a targeted drug delivery system would be that metabolism, adsorption, excretion or modification of the drug, can be avoided as the drug is protected by the "delivery vehicle".

There is a paucity of efficient drug delivery systems that offer increased drug half-life, bioavailability, selectivity, targeting and sustained release of therapeutic drugs.

The promise of highly specific and selective drugs based on monoclonal antibodies, although a class leader in the treatment of cancer, has fallen short of their initial promise. Although much more selective than the early oncology drugs, the efficiency is left wanting. Despite specific interaction e.g. monoclonal antibodies specifically interacting with a receptor the side effect profile is also often problematic. There is still an imbalance between tolerability and efficacy and therefore a significant reduction in quality of life through and after the therapeutic intervention.

The field of nanotechnology has opened up new avenues for medical science, especially in the field of targeted drug delivery. Conventional drug delivery methods have been based on liposomes/lipids, polymers of synthetic and natural origin, and inorganic nanoparticles. Nanoparticles used in drug delivery applications must be biocompatible with minimal toxicity. However, experience has shown that synthetic carriers are limited due to toxicity issues and low delivery efficiency with limited selectivity. Additionally, within the blood system they may fall apart and the free drug may be exposed. There is a clear need for the development of "smart" drug targeting systems.

Viruses have evolved to evade the hosts' immune system by having stable structures that are able to survive environmental stress and catabolism, yet they are able to survive sufficiently long enough to result in a cellular uptake for the release of their genetic material. Historically studied for their effects as pathogens, viruses play a key role in biological systems as the most abundant biological entities on earth. Viruses specifically lacking their genomic nucleic acid material are widely used as emerging targeted drug delivery platforms in bio-nanotechnology.

Viruses and noninfectious virus-like nanoparticles (VLNPs), e.g. as reviewed in Nkanga et al. ("The pharmacology of plant virus nanoparticles." Virology 556 (2021): 39-61), represent the ideal delivery drug vectors due to their intrinsically nature derived symmetry, uniformity of size and shape, and precise assembly of hundreds of molecules into highly organised scaffolds. VLNPs are assembled specifically from the coat proteins of the original virus. These viruses exhibit a distinctive diversity of shapes and sizes, from simple helical and icosahedral forms to more complex structures, ranging between 20 and 750 nanometers. Due to the well-defined structure and self-assembling system, a large number of VLNPs have been engineered to function as constrained carriers, imaging agents, drug/gene delivery vehicles, and other nano-materials.

Viruses are regarded as naturally occurring nucleic acid carriers that protect and carry their genetic payload, and that is the main property exploited for drug delivery purposes. VLNPs are best known for their immunogenic properties, but the versatility of VLNPs would allow a wide variety of applications. Recently, they have found themselves at the center of investigation in vaccinology, drug delivery and gene therapy.

The term "VLNPs" has been in use for at least 40 years. This includes empty structures of viral origin, infectious or non-infectious viruses, interfering viral nanoparticles with some structural modifications and non-infectious, self-assembled gene products acquired from the expression of viral structural genes in heterologous systems.

Viral envelope subunits or protomers can be bound by covalent interactions such as disulphide bonds or electron sharing, or non-covalent bonds such as hydrogen bonds, ionic interactions or Van der Waals forces. Some VLNPs are formed in a calcium-dependent manner, whereby the disulphide bonds increase stability of calcium binding. Abundance of those ions strengthens the structure of the particle, while removal of them loosens the interactions between protomers resulting in decreased particle stability or its disintegration into individual protomers. The VLNPs display remarkable cell entry propensity, which stems from the fact that they are formed from functional viral proteins that are responsible for cell penetration by the virus.

Over 100 VLNPs originating from microbial, insect and mammalian viruses have been produced and characterized since the beginning of the 1980s, derived from 35 different virus families. Like naturally occurring viruses, VLNPs can be enveloped or non-enveloped, spherical or filamentous. VLNPs assemble spontaneously during the viral cycle or in heterologous systems during expression of virus structural protein. Depending on the complexity of the VLNPs, they can be obtained by expression in prokaryotic or eukaryotic expression systems from the suitable recombinant vectors, or formed in cell-free conditions. Moreover, they can be built from proteins of a single virus, or can present the proteins or peptides derived from a virus or cell on a platform derived from any other single virus, thus forming chimeric VLNPs.

Natural delivery carriers, particularly VLNPs-based carriers, offer some unique advantages due to their morphological uniformity, biocompatibility, water solubility, designer functionalization and high uptake efficiency. Further, ideal nanomedical approaches for drug delivery or imaging aim to utilize biological behaviors to develop smart nanosized cages with high stability, appropriate pharmacokinetics, cell-targeting, and efficient cell penetrability.

A major disadvantage of current VLNP's is their native structure stemming in most cases from viruses infecting mammalian cells. Therefore, inherently they may interact with various cells and tissues and they may be prone to clearance by the immune system especially under repeated application. Natural previous infection by viruses may strongly impair the use of VLNP's derived from these viruses. Therefore, these VLNP's are exclusively used for vaccination purposes rather than for drug delivery.

While there is no clinically approved insect or bacteriophage-based nanomedicine, several are undergoing preclinical development while some systems are poised to enter translational development. VLNPs-based nanotechnology platforms undergoing development for various nanomedical applications are tobacco mosaic virus (TMV), cowpea mosaic virus (CPMV), cowpea chlorotic mottle virus (CCMV), physalis mottle virus (PhMV), and potato virus X (PVX) amongst others. Notable are also the following bacteriophages: MS2, P22, Qβ and M13. These viruses range in size from 30 nm to over a micron and can be found in a variety of distinctive shapes. With advances in biochemistry and directed evolution techniques, continuous progress has been made in the development of viral nanocarriers for drug delivery, imaging, and theranostic applications, including therapeutics and diagnostics in biomedical applications. These include gene therapies; monotherapy and combination therapies against cancer; vaccines against infectious diseases, cancer and other diseases; nanocarriers for imaging modalities; and theranostics with pho-tothermal therapy (PTT).

Nevertheless, there is still a need for new and improved VLNPs, especially VLNPs that can be used as a platform for a variety of applications including as drug delivery systems. In particular, there is a need for VLNPs that allow for targeted drug delivery, e.g. to tumor cells or cells found in diseased state, e.g. chronic inflammation or metabolic disorders. It would further be desirable, if the specific target of the VLNPs could be easily modified. In addition, there is a need for VLNPs that can be easily produced in high quantities.

It is an object of the present invention to provide such VLNPs.

Therefore, the present invention provides a polypeptide comprising a capsid protein sequence consisting of eight core regions and seven loop modification regions,

wherein the eight core regions are defined, respectively, by amino acid positions M1 to S309, P319 to R328, T340 to T342, M349 to F352, W359 to F362, V368 to Q373, T383 to T387 and T392 to N644 in the amino acid sequence according to SEQ ID NO: 1,

wherein the eight core regions of the capsid protein sequence have at least 70 % sequence identity to the eight core regions of the amino acid sequence according to SEQ ID NO: 1 or SEQ ID NO: 2 at least in the range from R32 to N570,

wherein the seven loop modification regions are defined, respectively, by amino acid positions A310 to 1318, H329 to 1339, 1343 to T348, D353 to E358, A363 to D367, T374 to A382, and A388 to V391 in the amino acid sequence according to SEQ ID NO: 1,

wherein at least one loop modification region of the capsid protein sequence differs from the corresponding loop modification region of the amino acid sequences according to SEQ ID NO: 1 and SEQ ID NO: 2 at least in that the at least one loop modification region comprises

- a peptide insert sequence comprising at least 3 amino acids, so that the at least one loop modification region of the capsid protein sequence is extended with respect to the corresponding loop modification region of the amino acid sequences according to SEQ ID NO: 1 and SEQ ID NO: 2 by said number of amino acids; and/or
- a cysteine residue,

wherein the amino acid numbering corresponds to the amino acid sequence according to SEQ ID NO: 1.

The polypeptide according to the invention is derived from a family of VLNP derived from *Alphatetraviridae* (ATV). The capsid protein (CP) of ATV has previously been characterized in two significantly different T = 4 quasi-equivalent assembly states when expressed as VLNPs in a baculovirus system. The procapsid at pH 7 is round, porous, and approximately 450 Å in diameter. It converts, in vitro, to the capsid-vessel form at pH 5 and the capsid-vessel is sealed shut, shaped like an icosahedron, has a maximum diameter of 410 Å and undergoes an autocatalytic cleavage at residue 570. Residues 571-644, the gamma peptide, remain associated with the particle and are partially ordered.

In the course of the invention, it has been surprisingly found that certain regions of the amino acid sequence of the capsid protein of *Alphatetraviridae* can be modified, without losing the ability to express the protein and assemble it into VLNPs. The inventors identified seven such regions in the sequence, which are herein referred to as "loop modification regions", while the remaining parts of the amino acid sequence are referred to as "core regions". It was found that it is possible to insert additional peptide sequences (herein referred to as "peptide insert sequences") into these loop modification regions, or, as an alternative, to incorporate cysteine residues, which allow for conjugation to chemical compounds, for instance through maleimide chemistry. The invention thus makes it possible to decorate VLNPs with peptides or other chemical compounds, thereby endowing them with additional functionality. For instance, the VLNPs may be decorated with targeting peptides that specifically bind to certain cells or tissues, e.g. tumor cells or cells found in a diseased state, e.g. chronic inflammation or metabolic disorders. In combination with efficient loading of the VLNP, this allows for targeted delivery of a payload to these cells. Using the VLNP as a drug carrier thus enables targeted drug delivery and therapy.

This approach has significant advantages e.g. over antibody-conjugates used in cancer therapy. Conjugates of monoclonal antibodies and a specific tumor killing entity (e.g. Trastuzumab emtansine used in breast cancer) are able only to carry one toxic principle to the tumor cell. The efficacy is therefore strongly limited since the concentration necessary to kill a tumor cell may not be achievable in vivo. A VLNP according to the invention can have the inner space filled with several hundreds or thousands of molecules specifically delivered to a tumor cell.

A further advantage of the VLNPs and polypeptides according to the invention is that they derive from viruses that have insects as natural hosts. Specifically, moths and butterflies serve as natural hosts for *Alphatetraviridae.* Therefore, the inventive VLNPs are less likely to interact with mammalian cells and tissues and less prone to clearance by the mammalian immune system than VLNPs derived from viruses primarily infecting mammalian cells.

Yet a further advantage of the inventive VLNPs and polypeptides is their ease and efficiency of production. It was found that the polypeptides can be produced in large amounts even in bacterial cells by refolding from inclusion bodies. Moreover, the loop modification regions identified by the inventors were found to tolerate significant modifications, while still allowing efficient production in high quantities.

In the context of the invention, the "capsid protein of Al*phatetraviridae"* preferably refers to the amino acid sequence of the capsid protein of *Nudaurelia capensis omega virus* (NωV) as disclosed in Agrawal and Johnson ("Sequence and analysis of the capsid protein of Nudaurelia capensis ω virus, an insect virus with T= 4 icosahedral symmetry." Virology 190.2 (1992): 806-814) and as deposited under GenBank accession number S43937.1. Various studies on this virus and corresponding VLNPs, partially also including modification to the capsid protein sequence, have been reported, among others in Maree et al. ("Surface display of an internal His-tag on virus-like particles of Nudaurelia capensis ω virus (NωV) produced in a baculovirus expression system." Journal of Virological Methods 136.1-2 (2006): 283-288) and in WO 97/46666 A1.

The crystal structure of capsid protein of *Nudaurelia capensis omega virus* is known from Munshi et al. ("The 2.8 Å structure of a T= 4 animal virus and its implications for membrane translocation of RNA." J. Mol. Biol 261.1 (1996): 1-10) as well as from Helgstrand et al. ("The refined structure of Nudaurelia capensis ω Virus reveals control elements for a T= 4 capsid maturation." Virology 318.1 (2004): 192-203). According to Munshi et al., the initial gene product consists of 644 amino acid residues and is cleaved between residues N570 and F571 in the mature VLNP. The protein is composed of a helical inner domain (where the cleavage occurs) containing residues preceding and following a canonical, viral, eight-stranded β-sandwich that forms the contiguous shell. Inserted between two strands of the shell domain are 133 residues with an immunoglobulin c-type fold (starting at residue 280 and finishing at residue 413). With said immunoglobulin-like domain, Munshi et al. identified 12 β-strands (labelled A, B, C, D, E, F, F', F", G, H, I, J) and 11 loops connecting these β-strands (AB, BC, CD, DE, EF, FF', F' F", F" G, GH, HI, IJ) .

The present inventors found that the immunoglobulin-like domain identified by Munshi et al. lends itself to modification; specifically, the loops connecting the 12 β-strands. However, it was surprisingly found that not all of the loops are well suited for such modification. Particularly advantageous results were achieved when the loops CD (A310 to 1318), EF (H329 to 1339), FF' (1343 to T348), F'F" (D353 to E358), F"G (A363 to D367), GH (T374 to A382), and HI (A388 to V391) - and among these, especially the loops HI (A388 to V391) and CD (A310 to 1318) - were selected for modification. These loops correspond to the loop modification regions defined herein. All other loops identified by Mushi et al., which are less favorable in terms of tolerating modifications, as well as the β-strands of the immunoglobulin-like domain and all other protein domains are part of the core regions as defined herein.

Unless specified otherwise, the residue numbering as used herein for any capsid protein sequence preferably refers to the amino acid sequence of said capsid protein of *Nudaurelia capensis omega virus* as set forth in SEQ ID NO: 1: Capsid protein of *Nudaurelia capensis omega virus,* NωV (SEQ ID NO: 1):

```
  1 MDSNSASGKR RSRNVRIAAN TVNVAPKQRQ ARGRRARSRA NNIDNVTAAA QELGQSLDAN
 61 VITFPTNVAT MPEFRSWARG KLDIDQDSIG WYFKYLDPAG ATESARAVGE YSKIPDGLVK
121 FSVDAEIREI YNEECPTVSD ASIPLDGAQW SLSIISYPMF RTAYFAVANV DNKEISLDVT
181 NDLIVWLNNL ASWRDVVDSG QWFTFSDDPT WFVRIRVLHP TYDLPDPTEG LLRTCSDYRL
241 TYKSITCEAN MPTLVDQGFW IGGHYALTPI ATTQNAVEGS GFEHPFNVTR PGIAAGVTLT
301 WASMPPGGSA PSGDPAWIPD STTQFQWRHG GFDAPTGVIT YTIPRGYTMQ YFDTTTNEWN
361 GFANPDDVVT FGQTGGAAGT NATITITAPT VTLTILATTT SAANVINFRN LDAETTAASN
421 RSEVPLPPLT FGQTAPNNPK IEQTLVKDTL GSYLVHSKMR NPVFQLTPAS SFGAISFTNP
481 GFDRNLDLPG FGGIRDSLDV NMSTAVCHFR SLSKSCSIVT KTYQGWEGVT NVNTPFGQFA
541 HSGLLKNDEI LCLADDLATR LTGVYGATDN FAAAVLAFAA NMLTSVLKSE ATTSVIKELG
601 NQATGLANQG LARLPGLLAS IPGKIAARVR ARRDRRRAAR MNNN
```

The seven loop modification regions identified by the inventors are marked in bold in the above sequence. They are defined, respectively, by amino acid positions A310 to 1318, H329 to I339, I343 to T348, D353 to E358, A363 to D367, T374 to A382, and A388 to V391 of SEQ ID NO: 1. The remaining regions, which are herein referred to as "core regions", are defined by amino acid positions M1 to S309, P319 to R328, T340 to T342, M349 to F352, W359 to F362, V368 to Q373, T383 to T387 and T392 to N644 of SEQ ID NO: 1.

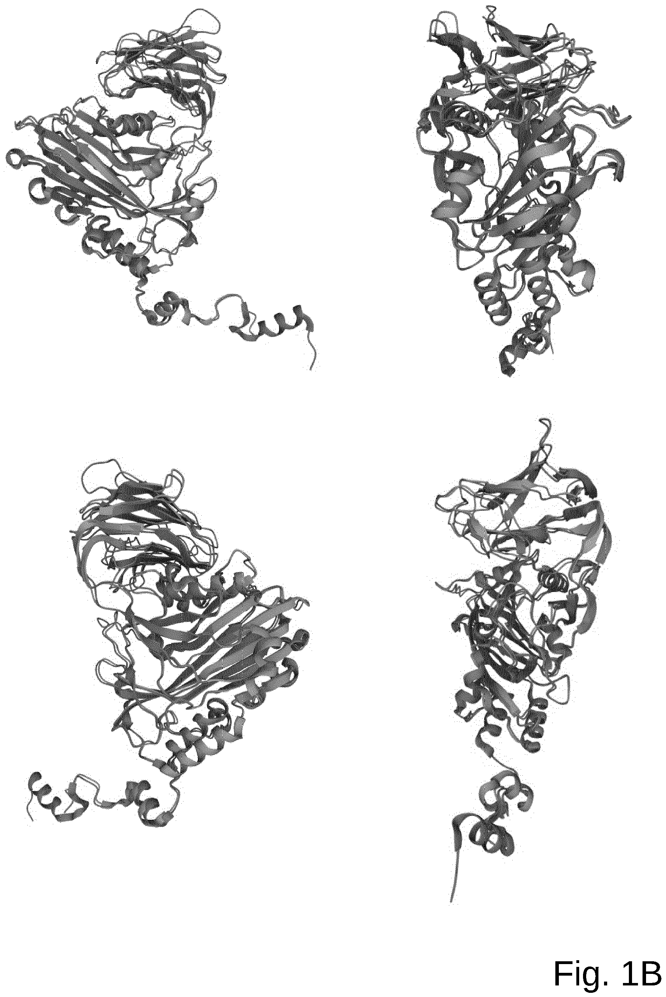

In the context of the invention, it is preferred if the inventive polypeptide is derived from the capsid protein of NωV, as described above. However, in a further preferred embodiment, the polypeptide is derived from the capsid protein of *Helicoverpa armigera stunt virus* (HaSV). HaSV and NωV are both members of *Alphatetraviridae* and have highly similar protein structures. A structural alignment, showing the close structural similarity, is shown in Fig. 1B. The preferred loop modification regions found in the context of the invention for the capsid protein of NωV therefore have corresponding preferred loop modification regions in the capsid protein of HaSV.

The sequence of the capsid protein of HaSV is disclosed in Hanzlik et al. ("Sequence of RNA2 of the Helicoverpa armigera stunt virus (Tetraviridae) and bacterial expression of its genes." Journal of General Virology 76.4 (1995): 799-811) and deposited under NCBI Reference Sequence NP_049237.1. In the context of the invention, the capsid protein of HaSV refers to the amino acid sequence as set forth in SEQ ID NO: 2:

Capsid protein of *Helicoverpa armigera stunt virus* (HaSV) (SEQ ID NO: 2):

```
  1 MGDAGVASQR PHNRRGTRNV RVSANTVTVN GRRNQRRRTG RQVSPPDNFT AAAQDLAQSL
 61 DANTVTFPAN ISSMPEFRNW AKGKIDLDSD SIGWYFKYLD PAGATESARA VGEYSKIPDG
121 LVKFSVDAEI REIYNEECPV VTDVSVPLDG RQWSLSIFSF PMFRTAYVAV ANVENKEMSL
181 DVVNDLIEWL NNLADWRYVV DSEQWINFTN DTTYYVRIRV LRPTYDVPDP TEGLVRTVSD
241 YRLTYKAITC EANMPTLVDQ GFWIGGQYAL TPTSLPQYDV SEAYALHTLT FARPSSAAAL
301 AFVWAGLPQG GTAPAGTPAW EQASSGGYLT WRHNGTTFPA GSVSYVLPEG FALERYDPND
361 GSWTDFASAG DTVTFRQVAV DEVVVTNNPA GGGSAPTFTV RVPPSNAYTN TVFRNTLLET
421 RPSSRRLELP MPPADFGQTV ANNPKIEQSL LKETLGCYLV HSKMRNPVFQ LTPASSFGAV
481 SFNNPGYERT RDLPDYTGIR DSFDQNMSTA VAHFRSLSHS CSIVTKTYQG WEGVTNVNTP
541 FGQFAHAGLL KNEEILCLAD DLATRLTGVY PATDNFAAAV SAFAANMLSS VLKSEATSSI
601 IKSVGETAVG AAQSGLAKLP GLLMSVPGKI AARVRARRAR RRAARAN
```

The loop modification regions are again marked in bold in the above sequence. The seven loop modification regions of the capsid protein of HaSV are defined by amino acid positions A313 to E321, H333 to V343, L347 to A352, D357 to S362, A367 to D371, T386 to G393 and A395 to F398 of SEQ ID NO: 2. The remaining regions are again referred to as the "core regions" and are defined by amino acid positions M1 to T312, Q322 to R332, S344 to V346, L353 to Y356, W363 to F366, T372 to V385, S394, and T399 to N647 of SEQ ID NO: 2. In the context of the present invention, the amino acid positions in any protein sequence may be referred to using the amino acid numbering that corresponds to the amino acid sequence according to SEQ ID NO: 1. In order to determine which position a given amino acid number refers to, the protein sequence in question is aligned to SEQ ID NO: 1, preferably following the procedure specified herein below for determining amino acid sequence identities, especially using the program "needle" of the EMBOSS software package using the parameters given herein below. If a certain amino acid position is given using the amino acid numbering corresponding to SEQ ID NO: 1, said given position designates the amino acid of the sequence in question that is matched to said given position of SEQ ID NO: 1 in the alignment. In case no amino acid is matched to said given position because the sequence in question has a gap in the alignment, the given amino acid position designates the first amino acid of the sequence in question preceding the gap. When a certain amino acid in the sequence in question has no matching amino acid in SEQ ID NO: 1 because SEQ ID NO: 1 has a gap in the alignment, it is not possible to refer to said specific amino acid in the sequence in question using the amino acid numbering corresponding to SEQ ID NO: 1.

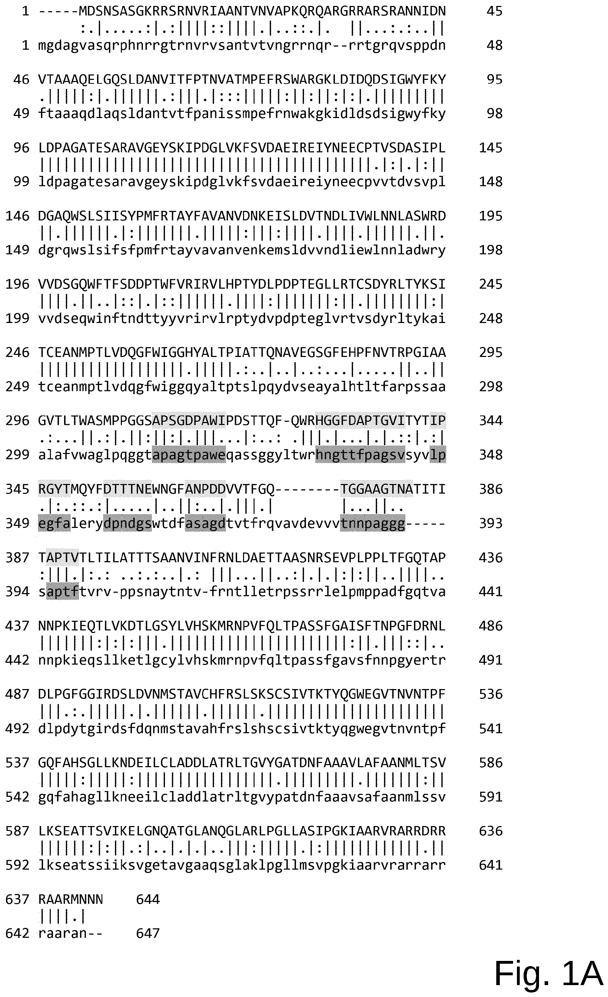

As an example, when referring to "T374 to A382 of the capsid protein of HaSV (SEQ ID NO: 2), wherein the amino acid numbering corresponds to the amino acid sequence according to SEQ ID NO:1", the designated positions in SEQ ID NO: 2 are determined by aligning the two sequences as shown in Fig. 1A. In this alignment, T374 of SEQ ID NO:1 is matched to T386 of SEQ ID NO: 2. Since no amino acid is matched to A382 of SEQ ID NO: 1, the amino acid preceding the gap in SEQ NO: 2 is used, which is G393. Thus, the language "T374 to A382 of SEQ ID NO: 2, wherein the amino acid numbering corresponds to the amino acid sequence according to SEQ ID NO: 1" is equal to "A382 to G393 of SEQ ID NO: 2".

Further examples for how gaps in the alignment are treated are given in the following, again with reference to Fig. 1A. "L396 of SEQ ID NO: 2, wherein the amino acid numbering corresponds to the amino acid sequence according to SEQ ID NO: 1" refers to V402 of SEQ ID NO: 2, because at this position the alignment contains a gap in SEQ ID NO: 2 and V402 is the amino acid in SEQ ID NO: 2 preceding this gap. However, V402 of SEQ ID NO: 2 may equally be referred to by "I395 of SEQ ID NO: 2, wherein the amino acid numbering corresponds to the amino acid sequence according to SEQ ID NO: 1", because I395 of SEQ ID NO: 1 is matched to V402 of SEQ ID NO: 2 in the alignment. Thus, both ways are equally valid for referring to V402 of SEQ ID NO: 2. On the other hand, as explained above, when a certain amino acid in the sequence in question has no matching amino acid in SEQ ID NO: 1 because SEQ ID NO: 1 has a gap in the alignment, it is not possible to refer to said specific amino acid in the sequence in question using the amino acid numbering corresponding to SEQ ID NO: 1. For instance, it is not possible to refer to L329 of SEQ ID NO: 2, because at this position the alignment contains a gap in SEQ ID NO: 1. However, it is of course possible to refer to Y328 of SEQ ID NO: 2 (by reference to F325 in the numbering according to SEQ ID NO: 1) and to T330 of SEQ ID NO: 2 (by reference to Q326 in the numbering according to SEQ ID NO: 1), because both of these amino acids have matching amino acids in SEQ ID NO: 1 in the alignment.

In the context of the invention, the loop modification regions of any given protein sequence are defined, respectively, by amino acid positions A310 to I318, H329 to I339, I343 to T348, D353 to E358, A363 to D367, T374 to A382, and A388 to V391, wherein the amino acid numbering corresponds to the amino acid sequence according to SEQ ID NO: 1. The core regions are defined as all regions of the protein sequence that are not part of a loop modification region. Thus, in order to determine the respective regions in a protein sequence in question, said sequence is aligned to SEQ ID NO: 1 and amino acid positions designated by the above definition are determined as laid out above. Fig. 1A shows, as an example, the loop modification regions of the capsid protein of HaSV (SEQ ID NO: 2) based on such an alignment. The loop modification regions are highlighted in Fig. 1A; the remaining regions (i.e., the core regions) are not highlighted. The same definitions apply to the loop modification regions and core regions of any polypeptide according to the invention.

All embodiments of the inventive polypeptides described herein with respect to SEQ ID NO: 1 are also preferred with respect to SEQ ID NO: 2. For instance, where a preferred embodiment specifies that the inventive polypeptide has a capsid protein sequence that has at least 70 % sequence identity to the amino acid sequence according to SEQ ID NO: 1 at least in the range from R32 to N570; this means that it is also a preferred embodiment that the inventive polypeptide has a capsid protein sequence that has at least 70 % sequence identity to the amino acid sequence according to SEQ ID NO: 2 at least in the range from R32 to N570, wherein the amino acid numbering corresponds to the amino acid sequence according to SEQ ID NO: 1. Based on the above definition, this latter embodiment can equally be defined as the inventive polypeptide having a capsid protein sequence that has at least 70 % sequence identity to the amino acid sequence according to SEQ ID NO: 2 at least in the range from R36 to N575.

The eight core regions of the capsid protein sequence have at least 70 % sequence identity to the eight core regions of the amino acid sequence according to SEQ ID NO: 1 at least in the range from R32 to N570. The sequence identity is determined as explained herein below. More specifically, it may be determined by aligning the amino acid sequence of the inventive polypeptide to the sequence according to SEQ ID NO: 1, preferably using the program "needle" of the EMBOSS software package using the parameters laid out herein below. Next the % sequence identity is calculated following the procedure laid out herein below, taking only into account those amino acid residues in SEQ ID NO: 1 that belong to core regions and that lie within the specified range of R32 to N570. This means that the number of amino acid residues scored as identical matches to residues belonging to core regions and lying within the range of R32 to N570 of SEQ ID NO: 1 is divided by the total number of residues belonging to core regions and lying within the range of R32 to N570 in SEQ ID NO: 1 and the result is multiplied by 100 to obtain the % sequence identity.

In the course of the invention, the inventors found that the loop modification regions of the capsid protein sequence tolerate significant modifications, while still allowing efficient production in high quantities, refolding to form functional protomers and assembly into the full VLNPs. Thus, in the inventive polypeptide, at least one loop modification region of the capsid protein sequence differs from the corresponding loop modification region of the amino acids sequence according to SEQ ID NO: 1. This difference may lie in that the at least one loop modification region comprises a peptide insert sequence comprising at least 3 amino acids, so that the at least one loop modification region of the capsid protein sequence is extended with respect to the corresponding loop modification region of the amino acid sequence according to SEQ ID NO: 1 by said number of amino acids. Outside of the peptide insert sequence, the loop modification region of the inventive polypeptide may be similar or identical to the corresponding loop modification region of the sequence according to SEQ ID NO: 1. Alternatively, the residues in the loop modification region of the sequence according to SEQ ID NO: 1 may also be mutated or replaced entirely by the peptide insert sequence, as long as the loop modification region of the inventive polypeptide is longer than the loop modification region of the sequence according to SEQ ID NO: 1 by said at least three amino acid residues. As an alternative or in addition to the peptide insert sequence, the difference may lie in that the at least one loop modification region comprises a cysteine residue. Also in this case, the remaining residues of the loop modification region of SEQ ID NO: 1 may be identical, mutated or even missing in the loop modification region of the inventive polypeptide.

In a preferred embodiment of the inventive polypeptide, the eight core regions of the capsid protein sequence have at least 70%, preferably at least 75%, preferably at least 80%, more preferably at least 85%, even more preferably at least 90%, even more preferably at least 92%, even more preferably at least 94%, even more preferably at least 95%, even more preferably at least 96%, even more preferably at least 97%, even more preferably at least 98%, yet even more preferably at least 99%, most preferably 100% sequence identity to the eight core regions of the amino acid sequence according to SEQ ID NO: 1 at least in the range from R32 to N570, preferably in the range of M1 to N570 or of R32 to N644, most preferably in the range of M1 to N644. In order of increasing preference, said sequence identity is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %.

It is further preferred, if the capsid protein sequence (i.e., the full capsid sequence consisting of the eight core regions and seven loop modification regions) has at least 70 %, preferably at least 75 %, more preferably at least 80%, even more preferably at least 85%, even more preferably at least 90%, even more preferably at least 92%, even more preferably at least 94%, even more preferably at least 95%, even more preferably at least 96%, even more preferably at least 97%, even more preferably at least 98%, most preferably at least 99% sequence identity to the amino acid sequence according to SEQ ID NO: 1 at least in the range from R32 to N570, preferably in the range of M1 to N570 or of R32 to N644, most preferably in the range of M1 to N644. In order of increasing preference, said sequence identity is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %. It is particularly preferred, if said sequence identity is in the ranges specified above (i.e., R32 to N570, preferably M1 to N644, etc.) but excluding the range of said at least one loop modification region of the capsid protein sequence that differs from the corresponding loop modification region of the amino acids sequence according to SEQ ID NO: 1. For instance, when said at least one loop modification region is the loop modification region defined by residues T374 to A382 in the amino acid sequence according to SEQ ID NO: 1, it is preferred if the specified sequence identity is in the range from R32 to N570 but excluding T374 to A382 (i.e., in the range from R32 to T374 and A382 to N570). When the polypeptide according to the invention comprises at least two loop modification regions that differ from the corresponding loop modification regions of the amino acids sequence according to SEQ ID NO: 1 as specified above, it is preferred that the specified sequence identity is in the ranges specified above but excluding the ranges of said at least two loop modification regions.

In the context of the invention, it is especially preferred, if the/each peptide insert sequence and/or the/each cysteine residue is located at a position within the capsid protein sequence selected from: G375 to A382, preferably A377 to T380; G330 to I339, preferably F332 to P335; N364 to D367, preferably P365; T354 to E358, preferably T355 to T356; P389 to V391, preferably P389 to T390; P344 to T348, preferably P344 to Y347; and/or P311 to I318, preferably S312 to D314. It has been found that these regions are particularly well suited for such modification. It was found to be particularly advantageous, when the peptide insert sequence and/or the cysteine residue is located at the position P389 to V391, preferably P389 to T390, within the capsid protein sequence. It was further found to be particularly advantageous, when the peptide insert sequence and/or the cysteine residue is located at the position P311 to I318, preferably S312 to D314, within the capsid protein sequence. In this context, "located at" a certain position is understood as the peptide insert sequence and/or the cysteine residue being inserted immediately before the indicated position; i.e., between the indicated amino acid residue and the preceding amino acid residue.

As shown in more detail in the Examples, it was found in the context of the invention that particularly advantageous results can be achieved, when the loops HI (A388 to V391) and CD (A310 to I318) are selected for modification. While all the loop modification as defined herein were found to advantageously lend themselves to modification, the loops HI and CD were found to give even better results than the other loops identified. The best results were obtained for the loop HI.

Thus, in a particularly preferred embodiment of the inventive polypeptide, the at least one loop modification region of the capsid protein sequence that differs from the corresponding loop modification region of the amino acids sequence according to SEQ ID NO: 1 is the loop modification region defined by residues A388 to V391 in the amino acid sequence according to SEQ ID NO: 1. It was found that this region lends itself particularly well to modification. In particular, this loop allows the insertion of a variety of peptide insert sequences, while still allowing efficient production in high quantities and effective display of the inserted peptide sequences (or chemical compounds conjugated to a cysteine residue) on VLNPs. In the context of this embodiment, it is particularly preferred if the capsid protein sequence has a sequence identity of at least 70 % to the amino acid sequence according to SEQ ID NO: 1 at least in the range from R32 to A388 and V391 to N570, preferably M1 to A388 and V391 to N570, or R32 to A388 and V391 to N644, most preferably M1 to A388 and V391 to N644. Preferably, the sequence identity is even higher, in order of increasing preference at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %. In the context of this embodiment, it is also preferred if at least one, especially at least two further loop modification regions differ from the corresponding loop modification region of the amino acids sequence according to SEQ ID NO: 1 as specified above.

In a particularly preferred embodiment of the inventive polypeptide, the at least one loop modification region of the capsid protein sequence that differs from the corresponding loop modification region of the amino acids sequence according to SEQ ID NO: 1 is the loop modification region defined by residues A310 to I318 in the amino acid sequence according to SEQ ID NO: 1. Also in this case, it was found that this region lends itself particularly well to modification. In the context of this embodiment, it is particularly preferred if the capsid protein sequence has a sequence identity of at least 70 % to the amino acid sequence according to SEQ ID NO: 1 at least in the range from R32 to A310 and I318 to N570, preferably M1 to A310 and I318 to N570, or R32 to A310 and I318 to N644, most preferably M1 to A310 and I318 to N644. Preferably, the sequence identity is even higher, in order of increasing preference at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %. Also in the context of this embodiment, it is particularly preferred if at least one, especially at least two further loop modification regions differ from the corresponding loop modification region of the amino acids sequence according to SEQ ID NO: 1 as specified above.

In a particularly preferred embodiment of the inventive polypeptide, the at least one loop modification region of the capsid protein sequence that differs from the corresponding loop modification region of the amino acids sequence according to SEQ ID NO: 1 is the loop modification region defined by residues T374 to A382 in the amino acid sequence according to SEQ ID NO: 1. Also in this case, it was found that this region lends itself particularly well to modification. In the context of this embodiment, it is particularly preferred if the capsid protein sequence has a sequence identity of at least 70 % to the amino acid sequence according to SEQ ID NO: 1 at least in the range from R32 to T374 and A382 to N570, preferably M1 to T374 and A382 to N570, or R32 to T374 and A382 to N644, most preferably M1 to T374 and A382 to N644. Preferably, the sequence identity is even higher, in order of increasing preference at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %. Also in the context of this embodiment, it is particularly preferred if at least one, especially at least two further loop modification regions differ from the corresponding loop modification region of the amino acids sequence according to SEQ ID NO: 1 as specified above.

In a particularly preferred embodiment of the inventive polypeptide, the at least one loop modification region of the capsid protein sequence that differs from the corresponding loop modification region of the amino acids sequence according to SEQ ID NO: 1 is the loop modification region defined by residues H329 to I339 in the amino acid sequence according to SEQ ID NO: 1. Also in this case, it was found that this region lends itself particularly well to modification. In the context of this embodiment, it is particularly preferred if the capsid protein sequence has a sequence identity of at least 70 % to the amino acid sequence according to SEQ ID NO: 1 at least in the range from R32 to H329 and I339 to N570, preferably M1 to H329 and I339 to N570, or R32 to H329 and I339 to N644, most preferably M1 to H329 and I339 to N644. Preferably, the sequence identity is even higher, in order of increasing preference at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %. Also in the context of this embodiment, it is particularly preferred if at least one, especially at least two further loop modification regions differ from the corresponding loop modification region of the amino acids sequence according to SEQ ID NO: 1 as specified above.

In a particularly preferred embodiment of the inventive polypeptide, the at least one loop modification region of the capsid protein sequence that differs from the corresponding loop modification region of the amino acids sequence according to SEQ ID NO: 1 is the loop modification region defined by residues A363 to D367 in the amino acid sequence according to SEQ ID NO: 1. Also in this case, it was found that this region lends itself particularly well to modification. In the context of this embodiment, it is particularly preferred if the capsid protein sequence has a sequence identity of at least 70 % to the amino acid sequence according to SEQ ID NO: 1 at least in the range from R32 to A363 and D367 to N570, preferably M1 to A363 and D367 to N570, or R32 to A363 and D367 to N644, most preferably M1 to A363 and D367 to N644. Preferably, the sequence identity is even higher, in order of increasing preference at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %. Also in the context of this embodiment, it is particularly preferred if at least one, especially at least two further loop modification regions differ from the corresponding loop modification region of the amino acids sequence according to SEQ ID NO: 1 as specified above.

In a particularly preferred embodiment of the inventive polypeptide, the at least one loop modification region of the capsid protein sequence that differs from the corresponding loop modification region of the amino acids sequence according to SEQ ID NO: 1 is the loop modification region defined by residues D353 to E358 in the amino acid sequence according to SEQ ID NO: 1. Also in this case, it was found that this region lends itself particularly well to modification. In the context of this embodiment, it is particularly preferred if the capsid protein sequence has a sequence identity of at least 70 % to the amino acid sequence according to SEQ ID NO: 1 at least in the range from R32 to D353 and E358 to N570, preferably M1 to D353 and E358 to N570, or R32 to D353 and E358 to N644, most preferably M1 to D353 and E358 to N644. Preferably, the sequence identity is even higher, in order of increasing preference at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %. Also in the context of this embodiment, it is particularly preferred if at least one, especially at least two further loop modification regions differ from the corresponding loop modification region of the amino acids sequence according to SEQ ID NO: 1 as specified above.

In a particularly preferred embodiment of the inventive polypeptide, the at least one loop modification region of the capsid protein sequence that differs from the corresponding loop modification region of the amino acids sequence according to SEQ ID NO: 1 is the loop modification region defined by residues I343 to T348 in the amino acid sequence according to SEQ ID NO: 1. Also in this case, it was found that this region lends itself particularly well to modification. In the context of this embodiment, it is particularly preferred if the capsid protein sequence has a sequence identity of at least 70 % to the amino acid sequence according to SEQ ID NO: 1 at least in the range from R32 to I343 and T348 to N570, preferably M1 to I343 and T348 to N570, or R32 to I343 and T348 to N644, most preferably M1 to I343 and T348 to N644. Preferably, the sequence identity is even higher, in order of increasing preference at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 %. Also in the context of this embodiment, it is particularly preferred if at least one, especially at least two further loop modification regions differ from the corresponding loop modification region of the amino acids sequence according to SEQ ID NO: 1 as specified above.

In a preferred embodiment of the inventive polypeptide at least two, preferably at least three, more preferably at least four, most preferably at least five of the seven loop modification regions of the capsid protein sequence comprise a peptide insert sequence comprising at least 3 amino acids, so that the loop modification region is extended with respect to the corresponding loop modification region of the amino acid sequence according to SEQ ID NO: 1 by said number of amino acids; and/or a cysteine residue. Preferably, in the polypeptide according to the invention at least one, two, three, four, five, six or seven; preferably one, two, three or four of the seven loop modification regions of the capsid protein sequence comprise a peptide insert sequence comprising at least 3 amino acids, so that the loop modification region is extended with respect to the corresponding loop modification region of the amino acid sequence according to SEQ ID NO: 1 by said number of amino acids; and/or a cysteine residue.

Preferably, the polypeptide according to the invention has the ability to self-assemble into VLNPs. The methods and conditions for self-assembly of VLNPs from the capsid protein of Al*phatetraviridae* are known in the art. In the context of the invention, the ability to self-assemble is preferably tested by refolding the polypeptide from inclusion bodies (IBs) and detecting the VLNPs by transmission electron microscopy (TEM), preferably as described in Example 1. The skilled person is familiar with how to perform the required experiments. For instance, an especially preferred method to test the ability to self-assemble is: providing the polypeptide in the form of IBs, preferably as described herein in Example 1; resuspending the IBs in 50mM Tris, pH 9 containing 4M urea; washing the IBs at 25°C in an orbital shaker at 120rpm for 1h; re-centrifuging the IBs at 50,000g for 1h at 25°C; adding 1g of washed IBs to 5ml of 8M urea, 50mM Tris pH9, 50mM DTT and incubating the mixture for 20 hours in an orbital shaker at 25°C; refolding and maturation in 150mM NaCl and 200mM Na Acetate); and detecting the presence of VLNPs by TEM. It is especially preferred if testing if the polypeptide has the ability to self-assemble into VLNPs is performed using expression, purification and characterization by TEM as described in Example 1.

The peptide insert sequence comprised in the at least one loop modification region preferably comprises at least 4, preferably at least 5, more preferably at least 6, more preferably at least 7, even more preferably at least 8, yet even more preferably at least 9, yet even more preferably at least 10, most preferably at least 15 amino acids. It is particularly preferred, if said peptide insert sequence comprises between 3 and 1000 amino acids, preferably between 4 and 500 amino acids, more preferably between 5 and 200 amino acids, even more preferably between 6 and 100 amino acids, yet even more preferably between 7 and 50 amino acids, yet even more preferably between 8 and 40 amino acids, yet even more preferably between 9 and 30 amino acids, most preferably between 10 and 25 amino acids.

When the at least one loop modification region comprises a cysteine residue, it is preferred that said cysteine residue is conjugated to a chemical compound, preferably a peptide, preferably via a maleimide-moiety. The skilled person is familiar with methods for conjugating chemical compounds to cysteine residues. A wide variety of sulfhydryl-reactive groups exist; for instance, haloacetyls, maleimides, aziridines, acryloyls, ary-lating agents, vinylsulfones, pyridyl disulfides, TNB-thiols and disulfide reducing agents. Most of these groups conjugate to sulfhydryls by either alkylation (usually the formation of a thioether bond) or disulfide exchange (formation of a disulfide bond). Such sulfhydryl reactive groups may simply be attached to the desired chemical compound, which can then be linked to the cysteine residue in the loop modification region.

The peptide insert sequences and/or chemical compounds may for instance be heterologous protein fragments or peptide sequences that may be derived from mammals (e.g., humans) or peptide sequences that may be derived from infectious agents, such as e.g., viruses, bacteria, fungi, parasites. The/each peptide insert sequence and/or the/each chemical compound conjugated to the/each cysteine residue preferably is a cell ligand, especially a cancer cell ligand. Particularly preferred are, for instance, tumor-associated antigens, tumor-specific antigens, tissue-specific antigens, cell type-specific antigens, and other antigens. In this way, VLNPs comprising the inventive polypeptide can be targeted to a wide variety of sites, tissues or cells *in vivo* and avoid interaction with other cells or tissues which do not express the respective target of the VLNP. In certain representations, the VLNPs described herein can be targeted to epithelial airway cells (lung), hepatocytes (liver), lymphocytes, and other tissues and/or cells.

In a preferred embodiment, the/each peptide insert sequence and/or the/each chemical compound is a receptor ligand, preferably selected from MC11, GE11 peptide, somatostatin.

In a further preferred embodiment the/each peptide insert sequence and/or the/each chemical compound is a receptor ligand, preferably a ligand of Integrin $\alpha v \beta 3$, SSTR2, GnRH-R, Bn receptors, VIP receptors, NTSR1, CCK2R, MC1R and/or hY1R. It is particularly preferred, if the/each peptide insert sequence and/or the/each chemical compound is selected from the group of peptides disclosed in Table 1 of Worm et al. ("Targeting of peptide-binding receptors on cancer cells with peptide-drug conjugates." Peptide Science 112.3 (2020): e24171), wherein the above-mentioned receptor ligands are also further described.

In a further preferred embodiment, the/each peptide insert sequence and/or the/each chemical compound is a peptide selected from the group consisting of Octreotide, RC160, Bombesin, PSAP-peptide, NT21MP, Nef-M1, Peptide R, Pentixafor, pHLIP, l-zipper peptide, ELP, $\alpha$-MSH mimics, GZP, cRGD, EETI 2.5 F (knottin), NGR, SP2012, AARP, CK, LyP-1, AGR, REA, LSD, iRGD, iPhage/pen, M2pep, CooP, CLT-1, Pep-1 L, Angiopep-2, Angiopep-7, FHK, tLyP-1, and/or Cilengitide. It is particularly preferred, if the/each peptide insert sequence and/or the/each chemical compound is selected from the group of peptides disclosed in Table 1 of Le Joncour et al. ("Seek & Destroy, use of targeting peptides for cancer detection and drug delivery." Bioorganic & Medicinal Chemistry 26.10 (2018): 2797-2806), wherein the above-mentioned peptides are also further described.

In a further preferred embodiment, the/each peptide insert sequence and/or the/each chemical compound is a peptide selected from the group consisting of CooP, ARF (1-22), ACooPK, T12, Angiopep-2, AP, RGD, RGD-4C, NGR, iNGR, cyclicRGD, Cilengitide, LyP-1, GE11, CTT, D-retroCTT, C6, G7-18NATE, UNO, TAT, Pene-tratin, iRGD, tLyP-1, PL3, Octreotide, $\alpha$-MSH, and/or Nle$^4$-D-Phe$^7$-$\alpha$-MSH$_{Oct}$. It is particularly preferred, if the/each peptide insert sequence and/or the/each chemical compound is selected from the group of peptides disclosed in Table 1 of Ayo et al. ("Peptide-Based Strategies for Targeted Tumor Treatment and Imaging." Pharmaceutics 13.4 (2021): 481), wherein the above-mentioned peptides are also further described.

The MC11-peptide (MQLPLATGGGC) has been shown to combine specially with fibroblast growth factor receptor (FGFR) on cell membranes. Decorating the VLNP with MC11 thus allows targeted receptor mediated delivery. FGF receptor-mediated delivery is e.g. described in Li et al. ("FGF receptor-mediated gene delivery using ligands coupled to polyethylenimine." Journal of Biomaterials Applications 22.2 (2007): 163-180).

The GE11-peptide (YHWYGYTPQNVI) is a ligand of epidermal growth factor receptor (ErbB1, EGFR). EGFR is overexpressed in a variety of human cancer cells. It has been considered as a rational target for drug delivery. GE11 has been shown to bind specifically and efficiently to EGFR, but with much lower mito-genic activity than EGF. GE11 internalizes preferentially into EGFR highly expressing cells, and they accumulate in EGFR over-expressing tumor xenografts after intravenous delivery in vivo (see e.g. Xu et al. "GE11 peptide-conjugated nanoliposomes to enhance the combinational therapeutic efficacy of docetaxel and siRNA in laryngeal cancers." International Journal of Nanomedicine 12 (2017): 6461). Thus, a VLNP carrying GE11 is attractive for targeted receptor-mediated drug delivery to cancer cells.

In a further preferred embodiment, the/each peptide insert sequence and/or the/each chemical compound is an anti-proliferative agent, preferably selected from antimetabolite agents, alkylating agents, antibiotic-type agents, hormonal anticancer agents, immunological agents, interferon-type agents, and/or antineoplastic agents.

Preferably, the/each peptide insert sequence and/or the/each chemical compound is an anticancer therapeutic agent, preferably selected from the group consisting of Taxol, Adriamycin, dactinomycin, bleomycin, vinblastine, cisplatin, acivicin; aclarubicin; acodazole hydro chloride; acronine; adozelesin; aldesleukin; altretamine; ambomycin; ametantrone acetate; amino-glutethimide; amsacrine; anastrozole; anthramycin; asparaginase; asperlin; azacitidine; azetepa; azotomycin; batimastat; benzo-depa; bicalutamide; bisantrene hydrochloride; bisnafide; dimesylate; bizelesin; bleomycin sulfate; brequinar sodium; bro-pirimine; busulfan; cactinomycin; calusterone; caracemide; car-betimer; carboplatin; carmustine; carubicin hydrochloride; car-zelesin; cedefingol; chlorambucil; cirolemycin; cladribine; crisnatol mesylate; cyclophosphamide; cytarabine; dacarbazine; daunorubicin hydrochloride; decitabine; dexormaplatin; dezaguanine; dezaguanine mesylate; diaziquone; doxorubicin; doxorubicin hydrochloride; droloxifene; droloxifene citrate; dromostanolone propionate; duazomycin; edatrexate; eflomithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; fluorocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alpha-2a; interferon alpha-2b; interferon alpha-n1; interferon alpha-n3; interferon beta I a, interferon gamma-I b;

iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopu rine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; temozolomide, teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and/or zorubicin hydrochloride.

When at least two loop modification regions of the capsid protein sequence differ from the corresponding loop modification region of the amino acids sequence according to SEQ ID NO: 1, different peptide insert sequences and/or a cysteine may be comprised in each of the differing loop modification regions. For instance, one loop modification region may comprise a peptide insert sequence as specified above, while a further loop modification region may comprise a cysteine residue. Alternatively, or in addition, two loop modification regions may comprise two different peptide insert sequences as specified above. It is particularly preferred, when at least two, especially at least three loop modification regions of the capsid protein sequence differ from the corresponding loop modification regions of the amino acids sequence according to SEQ ID NO: 1 at least in that each of said loop modification regions comprises a different peptide insert sequence as specified herein above. Thus, a different peptide insert sequence may be inserted into each differing loop modification region; e.g., a first peptide insert sequence in a first loop modification region, a second peptide insert sequence in a second loop modification region, and optionally a third targeting peptide in a third loop modification region. There may also be a combination in that a first peptide insert sequence is inserted in one or more loop modification regions and a second peptide insert sequence is inserted in one or more different loop modification regions, etc. wherein one or more of the peptide insert sequences independently may be included in two or more loop modification regions. In addition, one or more loop modification regions may include two or more peptide insert sequences (e.g. two or more copies of the same peptide insert sequence, or one or more each of two or more different peptide insert sequence) and/or one or more peptide insert sequences in combination with a cysteine residue.

Thus, in a preferred embodiment, at least two, preferably at least three, even more preferably at least four loop modification regions comprise the same peptide insert sequence and/or the same chemical compound conjugated to the cysteine residue. In a further preferred embodiment, the loop modification regions comprise at least two different peptide insert sequences and/or at least two different chemical compounds conjugated to the cysteine residue and/or at least one peptide insert sequence and at least one chemical compound conjugated to the cysteine residue.

The polypeptide according to the present invention may include further amino acid sequences before or after the capsid protein sequence. For instance, the polypeptide may further comprise, preferably N-terminally to the capsid protein sequence, a heterologous signal peptide, a cell penetrating peptide and/or a peptide for nuclear or cytoplasmic delivery.

In further aspects, the present invention provides a nucleic acid molecule comprising a sequence encoding the polypeptide according to the invention; an expression vector comprising the nucleic acid molecule according to the invention; a host cell comprising the expression vector according to the invention; and a method for producing the polypeptide according to the invention comprising culturing the host cell according to the preceding embodiment and recovering the polypeptide.

In yet a further aspect, the present invention provides a Virus-like Nanoparticle (VLNP) comprising the inventive polypeptide.

Preferably, the VLNP comprises several copies of the inventive polypeptide, preferably at least 2 copies, more preferably at least 5 copies, even more preferably at least 20 copies, yet even more preferably at least 50 copies, yet even more preferably at least 100 copies, yet even more preferably at least 150 copies, most preferably 240 copies.

However, the VLNP may also comprise different protomers; e.g., a mixture of polypeptides according to the invention and wild-type or non-modified capsid proteins. Preferably the mean proportion of protomers that are polypeptides according to the invention is between 1%(mole%; n/n) and 100%(n/n), preferably between 5%(n/n) and 90%(n/n), more preferably between 10%(n/n) and 80%(n/n), more preferably between 20%(n/n) and 70%(n/n), most preferably between 30%(n/n) and 60%(n/n). Having a mixture of non-modified and modified protomers allows to control the number of peptide insert sequences / chemical compounds displayed on the VLNP while at the same time ensuring particularly good and stable assembly. In addition, by controlling the number of targeting peptides or compounds displayed on each VLNP, it is possible to precisely fine-tune the affinity of the VLNP for its target. The average proportion of a chimeric VLNP may be determined by Western Blotting or sandwich ELISA, wherein the percentage corresponds to the relative signal intensity with respect to a VLNP comprising only the inventive polypeptide as protomers.

In a further preferred embodiment, the VLNP comprises at least a first polypeptide according to the invention and at least a second polypeptide according to the invention, wherein the first polypeptide differs from the second polypeptide. Preferably, the at least one loop modification region of the first polypeptide differs from the at least one loop modification region of the second polypeptide. It is especially preferred, if the first polypeptide comprises a first peptide insert sequence and the second polypeptide comprises a second peptide insert sequence that differs from the first peptide insert sequence. It is also preferred, if the first polypeptide comprises a first peptide insert sequence and the second polypeptide comprises a loop modification region comprising a cysteine residue. In a further preferred embodiment, the at least one loop modification region of the first polypeptide comprises a cysteine residue conjugated to a first chemical compound and the at least one loop modification region of the second polypeptide comprises a cysteine residue conjugated to a second chemical compound that differs from the first chemical compound. All preferred embodiments disclosed herein with respect to the inventive polypeptide (e.g. as regards the loop modification region, the peptide insert sequence, or the chemical compound) are also preferred, independently, for the first polypeptide and the second polypeptide.

Advantageously, in the context of producing the inventive VLNPs, the first polypeptide and the second polypeptide may be provided separately, preferably by expression in inclusion bodies, but be mixed before allowing the VLNP to spontaneously assemble from the polypeptide.

In this way, VLNPs are provided that may be decorated with two or more different peptides or other chemical compounds. Importantly, by controlling the ratio of modified protomers used to assemble the VLNPs, the ratio of these peptides or other chemical compounds on the VLNPs can be controlled. For instance, when two different targeting peptides or compounds are used, the affinity of the VLNP for each target can be tuned by increasing or decreasing the proportion of the respective modified protomer.

Preferably, the molar ratio between the first polypeptide and the second polypeptide is between 1:50 and 50:1, preferably between 1:20 and 20:1; more preferably between 1:10 and 10:1; even more preferably between 1:5 and 5:1; yet even more preferably between 1:2 and 2:1; especially between 1:1.5 and 1.5:1.

In a further preferred embodiment, the VLNP further comprises at least a third polypeptide according to the invention, wherein the third polypeptide differs from the first polypeptide and from the second polypeptide. All preferred embodiments disclosed herein for the first and the second polypeptide are also preferred in the context of the third polypeptide.

Preferably, the VLNPs according to the invention are non-aggregating. Non-aggregating in this context is preferably understood as the VLNPs forming separated particles when they are suspended in water at a concentration of 1 mg/mL, as determined by TEM.

The inventive VLNP preferably comprises a payload. Methods for loading plant virus-based VLNPs are known in the art, e.g. from Yildiz et al. ("Infusion of imaging and therapeutic molecules into the plant virus-based carrier cowpea mosaic virus: cargo-loading and delivery." Journal of Controlled Release 172.2 (2013): 568-578). In this way, by decorating the VLNP with the appropriate peptide insert sequences or chemical compounds as described above, the VLNP may be used to deliver a payload to a specific target site, tissue or cell in a subject.

Preferably, the payload is an active agent. Especially preferred are cytotoxic agents, preferably selected from 5-fluorouracil, leucovorin, capecitabine, cyclosphosphamide, docetaxel, placitaxel, gemcitabine, and/or platin-based drugs such as cisplatin or carboplatin. In this way, the inventive VLNP may be used for targeted cancer therapy.

However, targeted drug delivery may also highly advantageous in other treatments. For instance, in a further preferred embodiment, the payload may be an anti-infectious agent, preferably an antibacterial, an antiviral, an anti-parasitic and/or an antifungal agent and/or a radionucleotide. In yet a further preferred embodiment, the payload is an anti-inflammatory agent, preferably a COX-1 inhibitor.

In another embodiment, the payload may be a detectable marker, preferably a radionucleotide. This embodiment is particularly advantageous in the context of diagnostics and/or therapeutics. Targeted delivery by VLNP may allow high amounts of detectable markers to be delivered to specific targets, e.g. cancer cells, and thus allow for highly sensitive detection.

In further preferred embodiments, the payload may be a peptide or a protein. The payload may also be a natural or synthetic nucleic acid, in particular selected from the group consisting of nucleic acids encoding a desired protein such as mRNA, cDNA, a plasmid or vector, inhibitory nucleic acids such as siRNA or miRNA and/or nucleic acids having catalytic activity such as a ribozyme. Such embodiments are particularly useful when the VLNPs are used as vaccines.

Preferably, the VLNP comprises at least 1, preferably at least 2, more preferably at least 5, more preferably at least 10, even more preferably at least 20, even more preferably at least 50, even more preferably at least 100, yet even more preferably at least 200, yet even more preferably at least 500, most preferably at least 1000 molecules of the cargo. The number of molecules of the cargo comprised in the VLNP can be determined as described herein in the Examples. The skilled person is familiar with how to determine the number of cargo molecules in a VLNP; e.g., as described in Biabanikhankahdani et al. ("A simple add-and-display method for immobilisation of cancer drug on His-tagged virus-like nanoparticles for controlled drug delivery." Scientific Reports 7.1 (2017): 1-12).

In a preferred embodiment, the VLNP comprises at least two different payloads. Combining two different payloads within the same VLNP advantageously allows to deliver two, e.g. synergistic, payloads to a certain target. All preferred embodiments disclosed herein for the payload are also preferred, independently, for each of the at least two different payloads.

In a further aspect, the present invention provides a method for producing a VLNP according to the invention, comprising the steps of

- providing the polypeptide according to the invention in the form of inclusion bodies (IBs);
- solubilizing said IBs;
- refolding the polypeptide; and
- allowing the VLNP to spontaneously assemble from the polypeptide.

In order to obtain the IBs standard techniques known in the art can be used. The skilled person is familiar with methods to recombinantly express a certain polypeptide in suitable host cells, e.g. *E. coli,* and obtain IBs containing said polypeptide. Preferably, the polypeptide in the form of IBs is provided by the steps of: culturing host cells expressing a gene encoding the polypeptide; and obtaining IBs from said host cells. It is preferred that the host cells are prokaryotic cells, preferably *E. coli* cells.

Also for solubilizing the IBs and refolding the polypeptide, standard methods known in the art may be used. Preferably, the inclusion bodies are solubilized by transferring them into a solubilization buffer containing a denaturing agent (e.g., urea or guanidinium chloride) and preferably a reducing agent (e.g., dithiothreitol (DTT), β-Mercaptoethanol, TCEP (tris(2-carboxyethyl)phosphine) or cysteine). Refolding may then be achieved by removal of denaturant; e.g., by transferring the protein into a refolding buffer containing no or low concentrations of denaturing agents and/or reducing agents. Transferring into refolding buffer may be achieved, for instance, by dilution or by dialysis.

Finally, the skilled person is also able to choose appropriate conditions under which the polypeptides spontaneously assemble to form VLNPs. Such conditions are known in the art for VLNPs of *Alphatetraviridae* in general and may be used for the inventive VLNPs as well. In particular, the polypeptides may be subjected to higher $Ca^{2+}$-concentrations and/or oxidizing conditions. Suitable conditions are described herein in the examples.

In a preferred embodiment, the VLNP is allowed to spontaneously assemble from the polypeptide in the presence of a payload. In this way, VLNP comprising a payload may be conveniently be obtained. For example, mixing of the payload and the polypeptide may be performed under conditions where no or only limited VLNP assembly occurs, such as at low $Ca^{2+}$-concentrations and/or reducing conditions, and after addition of all desired components the conditions may be changed into those favorable for VLNP assembly, such as higher $Ca^{2+}$-concentrations and/or oxidizing conditions.

However, VLNP production may also occur in vivo. In particular, the components of the VLNPs may be co-expressed in a host cell and the VLNPs may assemble inside the host cell or upon lysis or disruption of the host cell.

In a preferred embodiment, the inventive method for producing a VLNP further comprises a step of purifying the VLNP, preferably the loaded VLNP, preferably by sucrose density gradient ultracentrifugation.

All preferred embodiments described herein with respect to the inventive VLNP and the payload are of course also preferred in the context of the inventive method for producing a VLNP.

In a further aspect, the present invention provides a composition comprising the inventive VLNP and a pharmaceutically acceptable excipient.

The excipient preferably is pharmaceutically acceptable for administration to an individual, especially a mammal, in particular a human. Suitable excipients are known to the person skilled in the art, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives. Other suitable excipients include any compound that does not induce the production of antibodies when administered to a patient that are harmful for the patient. Examples are well tolerable proteins, polysaccharides, polylactic acids, polyglycolic acid, polymeric amino acids and amino acid copolymers. The composition is preferably suitable for parenteral administration, in particular intravenous administration. The composition may be provided in injectable dosage unit form, e.g. as a solution, suspension or emulsion, formulated in conjunction with the above-defined pharmaceutically acceptable excipients. Advantageously, the composition comprises at least 0.01 mg, preferably at least 0.1 mg, more preferably at least 1 mg, even more preferably at least 5 mg, especially at least 10 mg of the polypeptide according to the invention. It is further preferred if the composition contains the inventive polypeptide in a concentration of at least 0.0001 % (weight per weight, w/w), preferably at least 0.001 % w/w, more preferably at least 0.01 % w/w, even more preferably at least 0.1 % w/w, yet even more preferably at least 1 % w/w, especially at least 10 % w/w.

Advantageously the composition is a solid composition (at 25 °C and at atmospheric pressure), preferably a lyophilized composition. In another preferred embodiment, the composition is a liquid composition (at 25 °C and at atmospheric pressure). Advantageously the composition contains the polypeptide according to the invention in a concentration of at least 0.001 mg/mL, preferably at least 0.01 mg/mL, more preferably at least 0.1 mg/mL, yet even more preferably at least 0.5 mg/mL, most preferably at least 2 mg/mL.

When the VLNP comprises a payload, it is preferred if said payload is fully encapsulated in the VLNPs. However, this does not exclude that the VLNPs additionally can be associated with the payload, e.g. by way of binding of the payload to outside structures of the vessel. Some molecules of the payload might even be incompletely encapsulated within the vessel. However, it is preferred if at least a part of the molecules of the payload is fully encapsulated in the vessel. Hence, preferably at least 1%(w/w), more preferably at least 2%(w/w), more preferably at least 5%(w/w), even more preferably at least 10%(w/w), even more preferably at least 20%(w/w) yet even more preferably at least 50%(w/w), yet even more preferably at least 70%(w/w), most preferably at least 90%(w/w) of the total amount of the payload present in the composition is encapsulated in the VLNPs. Preferably, the indicated percentages correspond to the entrapment efficiency (EE). The skilled person is familiar with methods to determine EE; e.g., as described in Masarapu et al. ("Physalis mottle virus-like particles as nanocarriers for imaging reagents and drugs." Biomacromolecules 18.12 (2017): 4141-4153). Preferably, the encapsulation efficiency is determined as described in the Examples.

Preferably, the composition according to the invention is adapted for oral, topical or parenteral; preferably subcutaneous, intramuscular, intravenous, transdermal, topical, mucosal, buccal, sublingual and/or intranasal administration; especially-intramuscular or intravenous administration.

In a further aspect, the present invention relates to the inventive composition for use in medicine.

Preferably the inventive composition is for use in the prevention or treatment of cancer, preferably wherein an effective amount of the composition is administered to a subject in need thereof.

The invention also provides a method for preventing or treating a disease, preferably cancer, comprising administering an effective amount of the composition to an individual in need thereof.

The composition may consist of an appropriate formulation and a therapeutically effective amount used for oral, topical or parenteral application for prevention or treatment of diseases especially various cancer types.

The phrases "parenteral administration" and "administered parenterally" are art-recognized terms and include modes of administration other than enteral and topical administration, such as injections, and include, without limitation, intratumoral, intravenous, intramuscular, intrapleural, intravascular, intra-pericardial, intraarterial, intrathecal, intracapsular, intraorbital, intracardiac, intradermal, intraperitoneal, transtracheal, subcutaneous, subcuticular, intra-articular, subcapsular, subarachnoid, intraspinal and intrasternal injection and infusion.

The phrases "systemic administration," "administered systemically," "peripheral administration" and "administered peripherally" as used herein mean the administration of a compound, agent or other material other than directly into a specific tissue, organ, or region of the subject being treated (e.g., tumor site), such that it enters the animal's system and, thus, is subject to metabolism and other like processes, for example, subcutaneous administration.

"Treating", as used herein, means ameliorating the effects of, or delaying, halting or reversing the progress of a disease or disorder. The word encompasses reducing the severity and/or frequency of a symptom of a disease or disorder.

Virus-like particles have widespread potential applications in various areas of medicine; e.g., as vaccines, for targeted drug delivery and for gene therapy; e.g., as described in Nooraei et al. ("Virus-like particles: preparation, immunogenicity and their roles as nanovaccines and drug nanocarriers." Journal of nanobiotechnology 19.1 (2021): 1-27). The inventive VLNP is particularly well suited for such purposes.

For example: A patient suffering from metastasing cancer, e.g. breast cancer with HER2pos characteristics will be treated by VLNP injection of VLNP being (i) modified as described above with the ligand representing the binding motif of Her2 (human epidermal growth factor receptor 2) and (ii) loaded with a doxorubicin. The treatment will be infusion or multiple injection. Therapy control will be performed by imaging technologies (tumor shrinking).

Another example useful for vaccination may be (i) the modification of the VLNP with individual ligands or combination thereof specific for dendritic cells ligands targeting e.g. cDC1 cells which express CD8A, CLEC9A, ITGAE, ITGAX, THBD (CD141), and XCR1), and (ii) loading of VLNP with an appropriate mRNA coding for the antigen for the specific desired immune response (e.g. COVID 19 S-protein). These VLNP can be applied by intradermal, subcutaneous or even oral route to induce a protective immune response.

The term "preventing" or "prevention" as used herein means to stop a disease state or condition from occurring in an individual completely or almost completely or at least to a (preferably significant) extent, especially when the individual is predisposed to such a risk of contracting a disease state or condition. However, these terms should not be interpreted as an absolute success in the sense that a subject can never develop an associated disease, reaction or condition but as the reduction of the chance of developing the disease, reaction or condition in a prophylactic treatment.

A "subject", as used therein, can be a human or non-human animal. Non-human animals include, for example, livestock and pets, such as ovine, bovine, porcine, canine, feline and murine mammals, as well as reptiles, birds and fish. Preferably, the subject is human.

In the context of the present invention, the subject is preferably known to be at risk of developing a disease, preferably cancer; e.g. because of genetic risk factors, family history or other risk factors.

The language "effective amount" or "therapeutically effective amount" refers to a sufficient amount of the composition used in the practice of the invention that is effective to provide effective treatment in a subject, depending on the compound being used. That result can be reduction and/or alleviation of the signs, symptoms, or causes of a disease or disorder, or any other desired alteration of a biological system. An appropriate therapeutic amount in any individual case may be determined by one of ordinary skill in the art using routine experimentation.

A "therapeutic" treatment is a treatment administered to a subject who exhibits signs of pathology of a disease or disorder for the purpose of diminishing or eliminating those signs.

A "prophylactic" or "preventive" treatment is a treatment administered to a subject who does not exhibit signs of a disease or disorder, or exhibits only early signs of the disease or disorder, for the purpose of decreasing the risk of developing pathology associated with the disease or disorder, for example decreasing the risk of developing pathology associated with a NY-ESO-l-expressing cancer. In some embodiments, use of an anticancer particle described herein in a preventive treatment provides immunoprotection.

As explained above, the inventive VLNPs may be used as a drug delivery system in order to transport a payload, e.g. a certain active agent, to a desired target in the body. For instance, the invention may be used to package and/or deliver a therapeutic agent that is useful to treat an infection, an inflammatory disorder, cancer and/or any other disease or disorder.

In some representations, the inventive VLNPs may be used to target a diagnostic, prophylactic or therapeutic substance to an adversely affected area in a subject, such as, for example a tumor.

Thus, in an aspect, the inventive composition is for use in the treatment of cancer. In a further aspect, the inventive composition is for use in the diagnosis of cancer. The present invention also provides methods for treating a subject having an adverse condition are provided, comprising administering to the subject one or more compositions described herein in an amount effective to treat the condition.

In some representations VLNP with or without a payload may be used as a vaccine to induce an immune response toward a specific pathogen or tumor or ameliorating a disease. The present invention therefore also relates to the inventive composition for use as a vaccine.

Details of the invention may be used to package and/or deliver a nucleic acid that may be used to silence the expression of one or more target genes. For example, methods and compositions of the invention may be used to deliver an siRNA, an antisense RNA, or any combination thereof.

In the context of the invention, the inventive composition can be administered to a patient via any suitable route. For example, the composition may be administered via one or more routes including but not limited to, subcutaneous, intramuscular, intravenous, transdermal, topical routes, and through mucosal layers such as via buccal, sublingual, and intranasal routes.

To facilitate the understanding of the invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

"Percent (%) amino acid sequence identity", "X% sequence identity" or "X% identical" (such as "70% sequence identity" or "70% identical") with respect to a reference polypeptide or protein sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2, Megalign (DNASTAR) or the "needle" pairwise sequence alignment application of the EMBOSS software package. Those skilled in the art can determine appropriate parameters for aligning sequences, including any algorithms needed to achieve maximal alignment over the full length of the sequences being compared. For purposes herein, however, % amino acid sequence identity values are calculated using the sequence alignment of the computer programme "needle" of the EMBOSS software package (publicly available from European Molecular Biology Laboratory; Rice et al., EMBOSS: the European Molecular Biology Open Software Suite, Trends Genet. 2000 Jun;16(6):276-7, PMID: 10827456).

The needle programme can be accessed under the web site http://www.ebi.ac.uk/Tools/psa/emboss_needle/ or downloaded for local installation as part of the EMBOSS package from http://emboss.sourceforge.net/. It runs on many widely-used UNIX operating systems, such as Linux. To align two protein sequences, the needle programme is preferably run with the following parameters:

Commandline: needle -auto -stdout -asequence SEQUENCE_FILE_A -bsequence SEQUENCE_FILE_B -datafile EBLOSUM62 -gapopen 10.0 - gapextend 0.5 -endopen 10.0 -endextend 0.5 -aformat3 pair -sprotein1 -sprotein2 (Align_format: pair Report_file: stdout)

The % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y,$$

where X is the number of amino acid residues scored as identical matches by the sequence alignment program needle in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. In cases where "the sequence of A is more than N% identical to the entire sequence of B", Y is the entire sequence length of B (i.e. the entire number of amino acid residues in B). In cases referring to a range within a sequence, e.g. where "the sequence of A has at least N% sequence identity to the sequence of B in the range from residue (i) to residue (ii)", only the part of the sequence falling within residues (i) to (ii) is considered for determining X and Y. Unless specifically stated otherwise, all % amino acid sequence identity values used herein are obtained as described in the immediately preceding paragraph using the needle computer program.

Unless specified otherwise, all parameters as used herein correspond to parameters at IUPAC SATP-conditions ("Standard Ambient Temperature and Pressure"), in particular a temperature of 25 °C and a pressure of 101.300 Pa.

Percentages (%) as used herein correspond to weight per volume (w/v) unless specified as weight per weight (w/w) or otherwise.

The present invention relates to the following preferred embodiments:

Embodiment 1. A polypeptide comprising a capsid protein sequence consisting of eight core regions and seven loop modification regions,

wherein the eight core regions are defined, respectively, by amino acid positions M1 to S309, P319 to R328, T340 to T342, M349 to F352, W359 to F362, V368 to Q373, T383 to T387 and T392 to N644 in the amino acid sequence according to SEQ ID NO: 1,

wherein the eight core regions of the capsid protein sequence have at least 70 % sequence identity to the eight core regions of the amino acid sequence according to SEQ ID NO: 1 or SEQ ID NO: 2 at least in the range from R32 to N570,

wherein the seven loop modification regions are defined, respectively, by amino acid positions A310 to I318, H329 to I339, I343 to T348, D353 to E358, A363 to D367, T374 to A382, and A388 to V391 in the amino acid sequence according to SEQ ID NO: 1,

wherein at least one loop modification region of the capsid protein sequence differs from the corresponding loop modification region of the amino acid sequences according to SEQ ID NO: 1 and SEQ ID NO: 2 at least in that the at least one loop modification region comprises

- a peptide insert sequence comprising at least 3 amino acids, so that the at least one loop modification region of the capsid protein sequence is extended with respect to the corresponding loop modification region of the amino acid sequences according to SEQ ID NO: 1 and SEQ ID NO: 2 by said number of amino acids; and/or
- a cysteine residue,

wherein the amino acid numbering corresponds to the amino acid sequence according to SEQ ID NO: 1.

Embodiment 2. The polypeptide according to the preceding embodiment, wherein the eight core regions of the capsid protein sequence have at least 70%, preferably at least 75%, preferably at least 80%, more preferably at least 85%, even more preferably at least 90%, even more preferably at least 92%, even more preferably at least 94%, even more preferably at least 95%, even more preferably at least 96%, even more preferably at least 97%, even more preferably at least 98%, yet even more preferably at least 99%, most preferably 100% sequence identity to the eight core regions of the amino acid sequence according to SEQ ID NO: 1 or SEQ ID NO: 2 at least in the range from R32 to N570, preferably in the range of M1 to N570 or of R32 to N644, most preferably in the range of M1 to N644, wherein the amino acid numbering corresponds to the amino acid sequence according to SEQ ID NO: 1.

Embodiment 3. The polypeptide according to any one of the preceding embodiments, wherein the capsid protein sequence has at least 70 %, preferably at least 75 %, more preferably at least 80%, even more preferably at least 85%, even more preferably at least 90%, even more preferably at least 92%, even more preferably at least 94%, even more preferably at least 95%, even more preferably at least 96%, even more preferably at least 97%, even more preferably at least 98%, most preferably at least 99% sequence identity to the amino acid sequence according to SEQ ID NO: 1 or SEQ ID NO: 2 at least in the range from R32 to N570, preferably in the range of M1 to N570 or of R32 to N644, most preferably in the range of M1 to N644, wherein the amino acid numbering corresponds to the amino acid sequence according to SEQ ID NO: 1.

Embodiment 4. The polypeptide according to any one of the preceding embodiments, wherein at least two, preferably at least three, more preferably at least four, most preferably at least five of the seven loop modification regions of the capsid protein sequence differ from the corresponding loop modification regions of the amino acid sequences according to SEQ ID NO: 1 and SEQ ID NO: 2 at least in that each of said loop modification regions comprise

- a peptide insert sequence comprising at least 3 amino acids, so that the loop modification region is extended with respect to the corresponding loop modification region of the amino acid sequences according to SEQ ID NO: 1 and SEQ ID NO: 2 by said number of amino acids; and/or
- a cysteine residue.

Embodiment 5. The polypeptide according to any one of the preceding embodiments, wherein one, two, three, four, five, six or seven; preferably one, two, three or four of the seven loop modification regions of the capsid protein sequence differ from the corresponding loop modification regions of the amino acid sequences according to SEQ ID NO: 1 and SEQ ID NO: 2 at least in that each of said loop modification regions comprises

- a peptide insert sequence comprising at least 3 amino acids, so that the loop modification region is extended with respect to the corresponding loop modification region of the amino acid sequences according to SEQ ID NO: 1 and SEQ ID NO: 2 by said number of amino acids; and/or
- a cysteine residue.

Embodiment 6. The polypeptide according to any one of the preceding embodiments, wherein the/each peptide insert sequence and/or the/each cysteine residue is located at a position within the capsid protein sequence selected from:

- G375 to A382, preferably A377 to T380;
- G330 to I339, preferably F332 to P335;
- N364 to D367, preferably P365;
- T354 to E358, preferably T355 to T356;
- P389 to V391, preferably P389 to T390;
- P344 to T348, preferably P344 to Y347; and/or
- P311 to I318, preferably S312 to D314,

wherein the amino acid numbering corresponds to the amino acid sequence according to SEQ ID NO: 1.

Embodiment 7. The polypeptide according to any one of the preceding embodiments, wherein the peptide insert sequence and/or the cysteine residue is located at the position P389 to V391, preferably P389 to T390, within the capsid protein sequence, wherein the amino acid numbering corresponds to the amino acid sequence according to SEQ ID NO: 1.

Embodiment 8. The polypeptide according to any one of the preceding embodiments, wherein the peptide insert sequence and/or the cysteine residue is located at the position P311 to I318, preferably S312 to D314, within the capsid protein sequence, wherein the amino acid numbering corresponds to the amino acid sequence according to SEQ ID NO: 1.

Embodiment 9. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequences according to SEQ ID NO: 1 and SEQ ID NO: 2 is/are selected from the loop modification regions defined by residues: A310 to I318; H329 to I339; I343 to T348; D353 to E358; A363 to D367; T374 to A382 and/or A388 to V391 in the amino acid sequence according to SEQ ID NO: 1 and SEQ ID NO: 2, wherein the amino acid numbering corresponds to the amino acid sequence according to SEQ ID NO: 1.

Embodiment 10. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequences according to SEQ ID NO: 1 and SEQ ID NO: 2 is/are selected from the loop modification regions defined by residues: A310 to I318; H329 to I339; D353 to E358; A363 to D367; T374 to A382 and/or A388 to V391 in the amino acid sequence according to SEQ ID NO: 1 and SEQ ID NO: 2, wherein the amino acid numbering corresponds to the amino acid sequence according to SEQ ID NO: 1.

Embodiment 11. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequences according to SEQ ID NO: 1 and SEQ ID NO: 2 is/are selected from the loop modification regions defined by residues: A310 to I318; H329 to I339; A363 to D367; T374 to A382 and/or A388 to V391 in the amino acid sequence according to SEQ ID NO: 1 and SEQ ID NO: 2, wherein the amino acid numbering corresponds to the amino acid sequence according to SEQ ID NO: 1.

Embodiment 12. The polypeptide according to any one of the preceding embodiments, wherein the at least one, preferably the at least two, more preferably the at least three, even more preferably the at least four loop modification region(s) of the capsid protein sequence that differ(s) from the corresponding loop modification region(s) of the amino acids sequences according to SEQ ID NO: 1 and SEQ ID NO: 2 is/are selected from the loop modification regions defined by residues: A310 to I318; H329 to I339; T374 to A382 and/or A388 to V391 in the amino acid sequence according to SEQ ID NO: 1 and SEQ ID NO: 2, wherein the amino acid numbering corresponds to the amino acid sequence according to SEQ ID NO: 1.

Embodiment 13. The polypeptide according to any one of the preceding embodiments, wherein the at least one, preferably the at least two, more preferably the at least three loop modification region(s) of the capsid protein sequence that differ(s) from the corresponding loop modification region(s) of the amino acids sequences according to SEQ ID NO: 1 and SEQ ID NO: 2 is/are selected from the loop modification regions defined by residues: A310 to I318; T374 to A382 and/or A388 to V391 in the amino acid sequence according to SEQ ID NO: 1 and SEQ ID NO: 2, wherein the amino acid numbering corresponds to the amino acid sequence according to SEQ ID NO: 1.

Embodiment 14. The polypeptide according to any one of the preceding embodiments, wherein the at least one, preferably the at least two loop modification region(s) of the capsid protein sequence that differ(s) from the corresponding loop modification region(s) of the amino acids sequences according to SEQ ID NO: 1 and SEQ ID NO: 2 is/are selected from the loop modification regions defined by residues: A310 to I318 and/or A388 to V391 in the amino acid sequence according to SEQ ID NO: 1 and SEQ ID NO: 2, wherein the amino acid numbering corresponds to the amino acid sequence according to SEQ ID NO: 1.

Embodiment 15. The polypeptide according to any one of the preceding embodiments, wherein the at least one loop modification region of the capsid protein sequence that differs from the corresponding loop modification region of the amino acids sequences according to SEQ ID NO: 1 and SEQ ID NO: 2 is the loop modification region defined by residues A310 to I318 in the amino acid sequence according to SEQ ID NO: 1 and SEQ ID NO: 2, wherein the amino acid numbering corresponds to the amino acid sequence according to SEQ ID NO: 1.

Embodiment 16. The polypeptide according to any one of the preceding embodiments, wherein the at least one loop modification region of the capsid protein sequence that differs from the corresponding loop modification region of the amino acids sequences according to SEQ ID NO: 1 and SEQ ID NO: 2 is the loop modification region defined by residues H329 to I339 in the amino acid sequence according to SEQ ID NO: 1 and SEQ ID NO: 2, wherein the amino acid numbering corresponds to the amino acid sequence according to SEQ ID NO: 1.

Embodiment 17. The polypeptide according to any one of the preceding embodiments, wherein the at least one loop modification region of the capsid protein sequence that differs from the corresponding loop modification region of the amino acids sequences according to SEQ ID NO: 1 and SEQ ID NO: 2 is the loop modification region defined by residues I343 to T348 in the amino acid sequence according to SEQ ID NO: 1 and SEQ ID NO: 2, wherein the amino acid numbering corresponds to the amino acid sequence according to SEQ ID NO: 1.

Embodiment 18. The polypeptide according to any one of the preceding embodiments, wherein the at least one loop modification region of the capsid protein sequence that differs from the corresponding loop modification region of the amino acids sequences according to SEQ ID NO: 1 and SEQ ID NO: 2 is the loop modification region defined by residues D353 to E358 in the amino acid sequence according to SEQ ID NO: 1 and SEQ ID NO: 2, wherein the amino acid numbering corresponds to the amino acid sequence according to SEQ ID NO: 1.

Embodiment 19. The polypeptide according to any one of the preceding embodiments, wherein the at least one loop modification region of the capsid protein sequence that differs from the corresponding loop modification region of the amino acids sequences according to SEQ ID NO: 1 and SEQ ID NO: 2 is the loop modification region defined by residues A363 to D367 in the amino acid sequence according to SEQ ID NO: 1 and SEQ ID NO: 2, wherein the amino acid numbering corresponds to the amino acid sequence according to SEQ ID NO: 1.

Embodiment 20. The polypeptide according to any one of the preceding embodiments, wherein the at least one loop modification region of the capsid protein sequence that differs from the corresponding loop modification region of the amino acids sequences according to SEQ ID NO: 1 and SEQ ID NO: 2 is the loop modification region defined by residues T374 to A382 in the amino acid sequence according to SEQ ID NO: 1 and SEQ ID NO: 2, wherein the amino acid numbering corresponds to the amino acid sequence according to SEQ ID NO: 1.

Embodiment 21. The polypeptide according to any one of the preceding embodiments, wherein the at least one loop modification region of the capsid protein sequence that differs from the corresponding loop modification region of the amino acids sequences according to SEQ ID NO: 1 and SEQ ID NO: 2 is the loop modification region defined by residues A388 to V391 in the amino acid sequence according to SEQ ID NO: 1 and SEQ ID NO: 2, wherein the amino acid numbering corresponds to the amino acid sequence according to SEQ ID NO: 1.

Embodiment 22. The polypeptide according to any one of the preceding embodiments, wherein at least the loop modification region defined by residues A310 to I318 in the amino acid sequences according to SEQ ID NO: 1 and SEQ ID NO: 2, and at least one of the further loop modification regions of the capsid protein sequence differ from the corresponding loop modification regions of the amino acid sequences according to SEQ ID NO: 1 and SEQ ID NO: 2 at least in that the loop modification region defined by residues A310 to I318 and the at least one further of the loop modification regions comprise, each individually,

- a peptide insert sequence comprising at least 3 amino acids, so that the at least one loop modification region of the capsid protein sequence is extended with respect to the corresponding loop modification region of the amino acid sequences according to SEQ ID NO: 1 and SEQ ID NO: 2 by said number of amino acids; and/or
- a cysteine residue,

wherein the amino acid numbering corresponds to the amino acid sequence according to SEQ ID NO: 1.

Embodiment 23. The polypeptide according to any one of the preceding embodiments, wherein at least the loop modification region defined by residues A388 to V391 in the amino acid sequences according to SEQ ID NO: 1 and SEQ ID NO: 2, and at least one of the further loop modification regions of the capsid protein sequence differ from the corresponding loop modification regions of the amino acid sequences according to SEQ ID NO: 1 and SEQ ID NO: 2 at least in that the loop modification region defined by residues A310 to I318 and the at least one further of the loop modification regions comprise, each individually,

- a peptide insert sequence comprising at least 3 amino acids, so that the at least one loop modification region of the capsid protein sequence is extended with respect to the corresponding loop modification region of the amino acid sequences according to SEQ ID NO: 1 and SEQ ID NO: 2 by said number of amino acids; and/or
- a cysteine residue,

wherein the amino acid numbering corresponds to the amino acid sequence according to SEQ ID NO: 1.

Embodiment 24. A polypeptide comprising a capsid protein sequence consisting of eight core regions and seven loop modification regions,

wherein the eight core regions are defined, respectively, by amino acid positions M1 to S309, P319 to R328, T340 to T342, M349 to F352, W359 to F362, V368 to Q373, T383 to T387 and T392 to N644 in the amino acid sequence according to SEQ ID NO: 1,
wherein the eight core regions of the capsid protein sequence have at least 70 % sequence identity to the eight core regions of the amino acid sequence according to SEQ ID NO: 1 at least in the range from R32 to N570,
wherein the seven loop modification regions are defined, respectively, by amino acid positions A310 to I318, H329 to I339, I343 to T348, D353 to E358, A363 to D367, T374 to A382, and A388 to V391 in the amino acid sequence according to SEQ ID NO: 1,
wherein at least one loop modification region of the capsid protein sequence differs from the corresponding loop modification region of the amino acids sequence according to SEQ ID NO: 1 at least in that the at least one loop modification region comprises

- a peptide insert sequence comprising at least 3 amino acids, so that the at least one loop modification region of the capsid protein sequence is extended with respect to the corresponding loop modification region of the amino acid sequence according to SEQ ID NO: 1 by said number of amino acids; and/or
- a cysteine residue.

Embodiment 25. The polypeptide according to the preceding embodiment, wherein the eight core regions of the capsid protein sequence have at least 70%, preferably at least 75%, preferably at least 80%, more preferably at least 85%, even more preferably at least 90%, even more preferably at least 92%, even more preferably at least 94%, even more preferably at least 95%, even more preferably at least 96%, even more preferably at least 97%, even more preferably at least 98%, yet even more preferably at least 99%, most preferably 100% sequence identity to the eight core regions of the amino acid sequence according to SEQ ID NO: 1 at least in the range from R32 to N570, preferably in the range of M1 to N570 or of R32 to N644, most preferably in the range of M1 to N644.

Embodiment 26. The polypeptide according to any one of the preceding embodiments, wherein the capsid protein sequence has at least 70 %, preferably at least 75 %, more preferably at least 80%, even more preferably at least 85%, even more preferably at least 90%, even more preferably at least 92%, even more preferably at least 94%, even more preferably at least 95%, even more preferably at least 96%, even more preferably at least 97%, even more preferably at least 98%, most preferably at least 99% sequence identity to the amino acid sequence according to SEQ ID NO: 1 at least in the range from R32 to N570, preferably in the range of M1 to N570 or of R32 to N644, most preferably in the range of M1 to N644.

Embodiment 27. The polypeptide according to any one of the preceding embodiments, wherein at least two, preferably at least three, more preferably at least four, most preferably at least five of the seven loop modification regions of the capsid protein sequence differ from the corresponding loop modification regions of the amino acids sequence according to SEQ ID NO: 1 at least in that each of said loop modification regions comprises

- a peptide insert sequence comprising at least 3 amino acids, so that the loop modification region is extended with respect to the corresponding loop modification region of the amino acid sequence according to SEQ ID NO: 1 by said number of amino acids; and/or
- a cysteine residue.

Embodiment 28. The polypeptide according to any one of the preceding embodiments, wherein one, two, three, four, five, six or seven; preferably one, two, three or four of the seven loop modification regions of the capsid protein sequence differ from the corresponding loop modification regions of the amino acids sequence according to SEQ ID NO: 1 at least in that each of said loop modification regions comprises

- a peptide insert sequence comprising at least 3 amino acids, so that the loop modification region is extended with respect to the corresponding loop modification region of the amino acid sequence according to SEQ ID NO: 1 by said number of amino acids; and/or
- a cysteine residue.

Embodiment 29. The polypeptide according to any one of the preceding embodiments, wherein the/each peptide insert sequence and/or the/each cysteine residue is located at a position within the capsid protein sequence selected from:

- G375 to A382, preferably A377 to T380;
- G330 to I339, preferably F332 to P335;
- N364 to D367, preferably P365;
- T354 to E358, preferably T355 to T356;
- P389 to V391, preferably P389 to T390;
- P344 to T348, preferably P344 to Y347; and/or
- P311 to I318, preferably S312 to D314.

Embodiment 30. The polypeptide according to any one of the preceding embodiments, wherein the peptide insert sequence and/or the cysteine residue is located at the position P389 to V391, preferably P389 to T390, within the capsid protein sequence.

Embodiment 31. The polypeptide according to any one of the preceding embodiments, wherein the peptide insert sequence and/or the cysteine residue is located at the position P311 to I318, preferably S312 to D314, within the capsid protein sequence.

Embodiment 32. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequence according to SEQ ID NO: 1 is/are selected from the loop modification regions defined by residues: A310 to I318; H329 to I339; I343 to T348; D353 to E358; A363 to D367; T374 to A382 and/or A388 to V391 in the amino acid sequence according to SEQ ID NO: 1.

Embodiment 33. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequence according to SEQ ID NO: 1 is/are selected from the loop modification regions defined by residues: A310 to I318; H329 to I339; D353 to E358; A363 to D367; T374 to A382 and/or A388 to V391 in the amino acid sequence according to SEQ ID NO: 1.

Embodiment 34. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequence according to SEQ ID NO: 1 is/are selected from the loop modification regions defined by residues: A310 to I318; H329 to I339; A363 to D367; T374 to A382 and/or A388 to V391 in the amino acid sequence according to SEQ ID NO: 1.

Embodiment 35. The polypeptide according to any one of the preceding embodiments, wherein the at least one, preferably the at least two, more preferably the at least three, even more preferably the at least four loop modification region(s) of the capsid protein sequence that differ(s) from the corresponding loop modification region(s) of the amino acids sequence according to SEQ ID NO: 1 is/are selected from the loop modification regions defined by residues: A310 to I318; H329 to I339; T374 to A382 and/or A388 to V391 in the amino acid sequence according to SEQ ID NO: 1.

Embodiment 36. The polypeptide according to any one of the preceding embodiments, wherein the at least one, preferably the at least two, more preferably the at least three loop modification region(s) of the capsid protein sequence that differ(s) from the corresponding loop modification region(s) of the amino acids sequence according to SEQ ID NO: 1 is/are selected from the loop modification regions defined by residues: A310 to I318; T374 to A382 and/or A388 to V391 in the amino acid sequence according to SEQ ID NO: 1.

Embodiment 37. The polypeptide according to any one of the preceding embodiments, wherein the at least one, preferably the at least two loop modification region(s) of the capsid protein sequence that differ(s) from the corresponding loop modification region(s) of the amino acids sequence according to SEQ ID NO: 1 is/are selected from the loop modification regions defined by residues: A310 to I318 and/or A388 to V391 in the amino acid sequence according to SEQ ID NO: 1.

Embodiment 38. The polypeptide according to any one of the preceding embodiments, wherein the at least one loop modification region of the capsid protein sequence that differs from the corresponding loop modification region of the amino acids sequence according to SEQ ID NO: 1 is the loop modification region defined by residues A310 to I318 in the amino acid sequence according to SEQ ID NO: 1.

Embodiment 39. The polypeptide according to any one of the preceding embodiments, wherein the at least one loop modification region of the capsid protein sequence that differs from the corresponding loop modification region of the amino acids sequence according to SEQ ID NO: 1 is the loop modification region defined by residues H329 to I339 in the amino acid sequence according to SEQ ID NO: 1.

Embodiment 40. The polypeptide according to any one of the preceding embodiments, wherein the at least one loop modification region of the capsid protein sequence that differs from the corresponding loop modification region of the amino acids sequence according to SEQ ID NO: 1 is the loop modification region defined by residues I343 to T348 in the amino acid sequence according to SEQ ID NO: 1.

Embodiment 41. The polypeptide according to any one of the preceding embodiments, wherein the at least one loop modification region of the capsid protein sequence that differs from the corresponding loop modification region of the amino acids sequence according to SEQ ID NO: 1 is the loop modification region defined by residues D353 to E358 in the amino acid sequence according to SEQ ID NO: 1.

Embodiment 42. The polypeptide according to any one of the preceding embodiments, wherein the at least one loop modification region of the capsid protein sequence that differs from the corresponding loop modification region of the amino acids sequence according to SEQ ID NO: 1 is the loop modification region defined by residues A363 to D367 in the amino acid sequence according to SEQ ID NO: 1.

Embodiment 43. The polypeptide according to any one of the preceding embodiments, wherein the at least one loop modification region of the capsid protein sequence that differs from the corresponding loop modification region of the amino acids sequence according to SEQ ID NO: 1 is the loop modification region defined by residues T374 to A382 in the amino acid sequence according to SEQ ID NO: 1.

Embodiment 44. The polypeptide according to any one of the preceding embodiments, wherein the at least one loop modification region of the capsid protein sequence that differs from the corresponding loop modification region of the amino acids sequence according to SEQ ID NO: 1 is the loop modification region defined by residues A388 to V391 in the amino acid sequence according to SEQ ID NO: 1.

Embodiment 45. The polypeptide according to any one of the preceding embodiments, wherein at least the loop modification region defined by residues A310 to 1318 in the amino acid sequence according to SEQ ID NO: 1, and at least one of the further loop modification regions of the capsid protein sequence differ from the corresponding loop modification regions of the amino acid sequence according to SEQ ID NO: 1 at least in that the loop modification region defined by residues A310 to 1318 and the at least one further of the loop modification regions comprise, each individually,

- a peptide insert sequence comprising at least 3 amino acids, so that the at least one loop modification region of the capsid protein sequence is extended with respect to the corresponding loop modification region of the amino acid sequence according to SEQ ID NO: 1 by said number of amino acids; and/or
- a cysteine residue.

Embodiment 46. The polypeptide according to any one of the preceding embodiments, wherein at least the loop modification region defined by residues A388 to V391 in the amino acid sequence according to SEQ ID NO: 1, and at least one of the further loop modification regions of the capsid protein sequence differ from the corresponding loop modification regions of the amino acid sequence according to SEQ ID NO: 1 at least in that the loop modification region defined by residues A310 to 1318 and the at least one further of the loop modification regions comprise, each individually,

- a peptide insert sequence comprising at least 3 amino acids, so that the at least one loop modification region of the capsid protein sequence is extended with respect to the corresponding loop modification region of the amino acid sequence according to SEQ ID NO: 1 by said number of amino acids; and/or
- a cysteine residue.

Embodiment 47. A polypeptide comprising a capsid protein sequence consisting of eight core regions and seven loop modification regions,

wherein the eight core regions are defined, respectively, by M1 to T312, Q322 to R332, S344 to V346, L353 to Y356, W363 to F366, T372 to V385, S394, and T399 to N647 in the amino acid sequence according to SEQ ID NO: 2,

wherein the eight core regions of the capsid protein sequence have at least 70 % sequence identity to the eight core regions of the amino acid sequence according to SEQ ID NO: 2 at least in the range from R36 to N575 of SEQ ID NO: 2,

wherein the seven loop modification regions are defined, respectively, by amino acid positions A313 to E321, H333 to V343, L347 to A352, D357 to S362, A367 to D371, T386 to G393 and A395 to F398 in the amino acid sequence according to SEQ ID NO: 2,

wherein at least one loop modification region of the capsid protein sequence differs from the corresponding loop modification region of the amino acids sequence according to SEQ ID NO: 2 at least in that the at least one loop modification region comprises

- a peptide insert sequence comprising at least 3 amino acids, so that the at least one loop modification region of the capsid protein sequence is extended with respect to the corresponding loop modification region of the amino acid sequence according to SEQ ID NO: 2 by said number of amino acids; and/or
- a cysteine residue.

Embodiment 48. The polypeptide according to the preceding embodiment, wherein the eight core regions of the capsid protein sequence have at least 70%, preferably at least 75%, preferably at least 80%, more preferably at least 85%, even more preferably at least 90%, even more preferably at least 92%, even more preferably at least 94%, even more preferably at least 95%, even more preferably at least 96%, even more preferably at least 97%, even more preferably at least 98%, yet even more preferably at least 99%, most preferably 100% sequence identity to the eight core regions of the amino acid sequence according to SEQ ID NO: 2 at least in the range from R36 to N575, preferably in the range of M1 to N575 or of R36 to N647, most preferably in the range of M1 to N647.

Embodiment 49. The polypeptide according to any one of the preceding embodiments, wherein the capsid protein sequence has at least 70 %, preferably at least 75 %, more preferably at least 80%, even more preferably at least 85%, even more preferably at least 90%, even more preferably at least 92%, even more preferably at least 94%, even more preferably at least 95%, even more preferably at least 96%, even more preferably at least 97%, even more preferably at least 98%, most preferably at least 99% sequence identity to the amino acid sequence according to SEQ ID NO: 2 at least in the range from R36 to N575, preferably in the range of M1 to N575 or of R36 to N647, most preferably in the range of M1 to N647.

Embodiment 50. The polypeptide according to any one of the preceding embodiments, wherein at least two, preferably at least three, more preferably at least four, most preferably at least five of the seven loop modification regions of the capsid protein sequence differ from the corresponding loop modification regions of the amino acids sequence according to SEQ ID NO: 2 at least in that each of said loop modification regions comprises

- a peptide insert sequence comprising at least 3 amino acids, so that the loop modification region is extended with respect to the corresponding loop modification region of the amino acid sequence according to SEQ ID NO: 2 by said number of amino acids; and/or
- a cysteine residue.

Embodiment 51. The polypeptide according to any one of the preceding embodiments, wherein one, two, three, four, five, six or seven; preferably one, two, three or four of the seven loop modification regions of the capsid protein sequence differ from the corresponding loop modification regions of the amino acids sequence according to SEQ ID NO: 2 at least in that each of said loop modification regions comprises

- a peptide insert sequence comprising at least 3 amino acids, so that the loop modification region is extended with respect to the corresponding loop modification region of the amino acid sequence according to SEQ ID NO: 2 by said number of amino acids; and/or
- a cysteine residue.

Embodiment 52. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequence according to SEQ ID NO: 2 is/are selected from the loop modification regions defined by residues: A313 to E321; H333 to V343; L347 to A352; D357 to S362; A367 to D371; T386 to G393 and/or A395 to F398 in the amino acid sequence according to SEQ ID NO: 2.

Embodiment 53. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequence according to SEQ ID NO: 2 is/are selected from the loop modification regions defined by residues: A313 to E321; H333 to V343; D357 to S362; A367 to D371; T386 to G393 and/or A395 to F398 in the amino acid sequence according to SEQ ID NO: 2.

Embodiment 54. The polypeptide according to any one of the preceding embodiments, wherein the at least one, preferably the at least two, more preferably the at least three, even more preferably the at least four, especially the at least five loop modification region(s) of the capsid protein sequence that differ(s) from the corresponding loop modification region(s) of the amino acids sequence according to SEQ ID NO: 2 is/are selected from the loop modification regions defined by residues: A313 to E321; H333 to V343; A367 to D371; T386 to G393 and/or A395 to F398 in the amino acid sequence according to SEQ ID NO: 2.

Embodiment 55. The polypeptide according to any one of the preceding embodiments, wherein the at least one, preferably the at least two, more preferably the at least three, even more preferably the at least four loop modification region(s) of the capsid protein sequence that differ(s) from the corresponding loop modification region(s) of the amino acids sequence according to SEQ ID NO: 2 is/are selected from the loop modification regions defined by residues: A313 to E321; H333 to V343; T386 to G393 and/or A395 to F398 in the amino acid sequence according to SEQ ID NO: 2.

Embodiment 56. The polypeptide according to any one of the preceding embodiments, wherein the at least one, preferably the at least two, more preferably the at least three loop modification region(s) of the capsid protein sequence that differ(s) from the corresponding loop modification region(s) of the amino acids sequence according to SEQ ID NO: 2 is/are selected from the loop modification regions defined by residues: A313 to E321; T386 to G393 and/or A395 to F398 in the amino acid sequence according to SEQ ID NO: 2.

Embodiment 57. The polypeptide according to any one of the preceding embodiments, wherein the at least one, preferably the at least two loop modification region(s) of the capsid protein sequence that differ(s) from the corresponding loop modification region(s) of the amino acids sequence according to SEQ ID NO: 2 is/are selected from the loop modification regions defined by residues: A313 to E321 and/or A395 to F398 in the amino acid sequence according to SEQ ID NO: 2.

Embodiment 58. The polypeptide according to any one of the preceding embodiments, wherein the at least one loop modification region of the capsid protein sequence that differs from the corresponding loop modification region of the amino acids sequence according to SEQ ID NO: 2 is the loop modification region defined by residues A313 to E321 in the amino acid sequence according to SEQ ID NO: 2.

Embodiment 59. The polypeptide according to any one of the preceding embodiments, wherein the at least one loop modification region of the capsid protein sequence that differs from the corresponding loop modification region of the amino acids sequence according to SEQ ID NO: 2 is the loop modification region defined by residues H333 to V343 in the amino acid sequence according to SEQ ID NO: 2.

Embodiment 60. The polypeptide according to any one of the preceding embodiments, wherein the at least one loop modification region of the capsid protein sequence that differs from the corresponding loop modification region of the amino acids sequence according to SEQ ID NO: 2 is the loop modification region defined by residues L347 to A352 in the amino acid sequence according to SEQ ID NO: 2.

Embodiment 61. The polypeptide according to any one of the preceding embodiments, wherein the at least one loop modification region of the capsid protein sequence that differs from the corresponding loop modification region of the amino acids sequence according to SEQ ID NO: 2 is the loop modification region defined by residues D357 to S362 in the amino acid sequence according to SEQ ID NO: 2.

Embodiment 62. The polypeptide according to any one of the preceding embodiments, wherein the at least one loop modification region of the capsid protein sequence that differs from the corresponding loop modification region of the amino acids sequence according to SEQ ID NO: 2 is the loop modification region defined by residues A367 to D371 in the amino acid sequence according to SEQ ID NO: 2.

Embodiment 63. The polypeptide according to any one of the preceding embodiments, wherein the at least one loop modification region of the capsid protein sequence that differs from the corresponding loop modification region of the amino acids sequence according to SEQ ID NO: 2 is the loop modification region defined by residues T386 to G393 in the amino acid sequence according to SEQ ID NO: 2.

Embodiment 64. The polypeptide according to any one of the preceding embodiments, wherein the at least one loop modification region of the capsid protein sequence that differs from the corresponding loop modification region of the amino acids sequence according to SEQ ID NO: 2 is the loop modification region defined by residues A395 to F398 in the amino acid sequence according to SEQ ID NO: 2.

Embodiment 65. The polypeptide according to any one of the preceding embodiments, wherein at least the loop modification region defined by residues A313 to E321 in the amino acid sequence according to SEQ ID NO: 2, and at least one of the further loop modification regions of the capsid protein sequence differ from the corresponding loop modification regions of the amino acid sequence according to SEQ ID NO: 2 at least in that the loop modification region defined by residues A313 to E321 and the at least one further of the loop modification regions comprise, each individually,

- a peptide insert sequence comprising at least 3 amino acids, so that the at least one loop modification region of the capsid protein sequence is extended with respect to the corresponding loop modification region of the amino acid sequence according to SEQ ID NO: 2 by said number of amino acids; and/or
- a cysteine residue.

Embodiment 66. The polypeptide according to any one of the preceding embodiments, wherein at least the loop modification region defined by residues A395 to F398 in the amino acid sequence according to SEQ ID NO: 2, and at least one of the further loop modification regions of the capsid protein sequence differ from the corresponding loop modification regions of the amino acid sequence according to SEQ ID NO: 2 at least in that the loop modification region defined by residues A313 to E321 and the at least one further of the loop modification regions comprise, each individually,

- a peptide insert sequence comprising at least 3 amino acids, so that the at least one loop modification region of the capsid protein sequence is extended with respect to the corresponding loop modification region of the amino acid sequence according to SEQ ID NO: 2 by said number of amino acids; and/or
- a cysteine residue.

Embodiment 67. The polypeptide according to any one of the preceding embodiments, wherein the polypeptide has the ability to self-assemble into Virus-like Nanoparticles (VLNPs).

Embodiment 68. The polypeptide according to any one of the preceding embodiments, wherein the/each peptide insert sequence comprises at least 4, preferably at least 5, more preferably at least 6, more preferably at least 7, even more preferably at least 8, yet even more preferably at least 9, yet even more preferably at least 10, most preferably at least 15 amino acids.

Embodiment 69. The polypeptide according to any one of the preceding embodiments, wherein the/each peptide insert sequence comprises between 3 and 1000 amino acids, preferably between 4 and 500 amino acids, more preferably between 5 and 200 amino acids, even more preferably between 6 and 100 amino acids, yet even more preferably between 7 and 50 amino acids, yet even more preferably between 8 and 40 amino acids, yet even more preferably between 9 and 30 amino acids, most preferably between 10 and 25 amino acids.

Embodiment 70. The polypeptide according to any one of the preceding embodiments, wherein the cysteine residue is conjugated to a chemical compound, preferably a peptide, preferably via a maleimide-moiety.

Embodiment 71. The polypeptide according to any one of the preceding embodiments, wherein the/each peptide insert sequence and/or the/each chemical compound is a cell ligand, preferably a cancer cell ligand.

Embodiment 72. The polypeptide according to any one of the preceding embodiments, wherein the/each peptide insert sequence and/or the/each chemical compound is selected from tumor-associated antigens, tumor-specific antigens, tissue-specific antigens, cell type-specific antigens, and other antigens.

Embodiment 73. The polypeptide according to any one of the preceding embodiments, wherein the/each peptide insert sequence and/or the/each chemical compound is a receptor ligand, preferably selected from MC11, GE11 peptide and/or somatostatin.

Embodiment 74. The polypeptide according to any one of the preceding embodiments, wherein at least two, preferably at least three, even more preferably at least four loop modification regions comprise the same peptide insert sequence and/or the same chemical compound conjugated to the cysteine residue.

Embodiment 75. The polypeptide according to any one of the preceding embodiments, wherein the loop modification regions comprise at least two different peptide insert sequences and/or at least two different chemical compounds conjugated to the cysteine residue and/or at least one peptide insert sequence and at least one chemical compound conjugated to the cysteine residue.

Embodiment 76. The polypeptide according to any one of the preceding embodiments, wherein the polypeptide further comprises, preferably N-terminally to the capsid protein sequence, a heterologous signal peptide, a cell penetrating peptide and/or a peptide for nuclear or cytoplasmic delivery.

Embodiment 77. A nucleic acid molecule comprising a sequence encoding the polypeptide according to any one of the preceding embodiments.

Embodiment 78. An expression vector comprising the nucleic acid molecule according to the preceding embodiment.

Embodiment 79. A host cell comprising the expression vector according to the preceding embodiment.

Embodiment 80. A method for producing the polypeptide according to any one of the preceding embodiments comprising culturing the host cell according to the preceding embodiment and recovering the polypeptide.

Embodiment 81. A Virus-like Nanoparticle (VLNP) comprising the polypeptide according to any one of the preceding embodiments.

Embodiment 82. The VLNP according to the preceding embodiment, wherein the VLNP comprises at least a first polypeptide according to any one of the preceding embodiments, and at least a second polypeptide according to any one of the preceding embodiments, wherein the first polypeptide differs from the second polypeptide.

Embodiment 83. The VLNP according to the preceding embodiment, wherein the molar ratio between the first polypeptide and the second polypeptide is between 1:50 and 50:1, preferably between 1:20 and 20:1; more preferably between 1:10 and 10:1; even more preferably between 1:5 and 5:1; yet even more preferably between 1:2 and 2:1; especially between 1:1.5 and 1.5:1.

Embodiment 84. The VLNP according to any one of the preceding embodiments, wherein the VLNP comprises at least a third polypeptide according to any one of the preceding embodiments.

Embodiment 85. The VLNP according to any one of the preceding embodiments, wherein the VLNP comprises a payload.

Embodiment 86. The VLNP according to the preceding embodiment, wherein the payload is an active agent.

Embodiment 87. The VLNP according to any one of the preceding embodiments, wherein the payload is a cytotoxic agent, preferably selected from 5-fluorouracil, leucovorin, capecitabine, cy-closphosphamide, docetaxel, placitaxel, gemcitabine, and/or platin-based drugs such as cisplatin or carboplatin.

Embodiment 88. The VLNP according to any one of the preceding embodiments, wherein the payload is an anti-infectious agent, preferably an antibacterial, an antiviral, an anti-parasitic and/or an antifungal agent.

Embodiment 89. The VLNP according to any one of the preceding embodiments, wherein the payload is an anti-inflammatory agent, preferably a COX-1 inhibitor.

Embodiment 90. The VLNP according to any one of the preceding embodiments, wherein the payload is a detectable marker, preferably a radionucleotide.

Embodiment 91. The VLNP according to any one of the preceding embodiments, wherein the payload is peptide or a protein.

Embodiment 92. The VLNP according to any one of the preceding embodiments, wherein the payload is a natural or synthetic nucleic acid, in particular selected from the group consisting of nucleic acids encoding a desired protein such as mRNA, cDNA, a plasmid or vector, inhibitory nucleic acids such as siRNA or miRNA and/or nucleic acids having catalytic activity such as a ribozyme.

Embodiment 93. The VLNP according to any one of the preceding embodiments, wherein the payload is fully encapsulated in the VLNP.

Embodiment 94. The VLNP according to any one of the preceding embodiments, wherein the VLNP comprises at least two different payloads, wherein each payload is independently defined as in any one of the preceding embodiments.

Embodiment 95. A method for producing a VLNP according to any one of the preceding embodiments, comprising the steps of

- providing the polypeptide according to any one of the previous embodiments in the form of inclusion bodies (IBs);
- solubilizing said IBs;
- refolding the polypeptide; and
- allowing the VLNP to spontaneously assemble from the polypeptide.

Embodiment 96. The method for producing a VLNP according to the preceding embodiment, wherein the VLNP is allowed to spontaneously assemble from the polypeptide in the presence of a payload.

Embodiment 97. The method for producing a VLNP according to any one of the preceding embodiments, further comprising a step of purifying the VLNP, preferably by sucrose density gradient ultracentrifugation.

Embodiment 98. A composition comprising the VLNP according to any one of the preceding embodiments and a pharmaceutically acceptable excipient.

Embodiment 99. The composition according to the preceding embodiment, wherein the composition comprises a first VLNP according to any one of the preceding embodiments and at least one further VLNP, wherein the at least one further VLNP differs from the first VLNP.

Embodiment 100. The composition according to the preceding embodiment, wherein the at least one further VLNP is as defined in any one of the preceding embodiments.

Embodiment 101. The composition according to any one of the preceding embodiments, wherein the composition is adapted for subcutaneous, intramuscular, intravenous, transdermal, topical, mucosal, buccal, sublingual and/or intranasal administration; preferably intramuscular or intravenous administration.

Embodiment 102. The composition according to any one of the preceding embodiments, wherein the composition comprises between 0,01 and 500 mg/mL, preferably between 0,1 and 100 mg/mL, more preferably between 0,5 and 20 mg/mL of the VLNP.

Embodiment 103. The composition according to any one of the preceding embodiments for use in medicine.

Embodiment 104. The composition according to any one of the preceding embodiments for use in the treatment of cancer.

Embodiment 105. The composition for use according to any one of the preceding embodiments, wherein the composition is for use as a vaccine.

Embodiment 106. The composition for use according to any one of the preceding embodiments, wherein the composition is used for gene therapy.

Embodiment 107. The composition for use according to any one of the preceding embodiments, wherein the composition is used for targeted drug delivery.

Embodiment 108. The composition for use according to any one of the preceding embodiments, wherein the composition is used as standalone therapy.

Embodiment 109. The composition for use according to any one of the preceding embodiments, wherein the composition is used in combination with other suitable therapeutics.

Embodiment 110. The composition according to any one of the preceding embodiments for use in the diagnosis of cancer.

The present invention is further illustrated by the following figures and examples, without being limited thereto.

**Figure 1.** (A) Sequence alignment of capsid protein sequence of NωV according to SEQ ID NO: 1 (shown as uppercase letters) and of HaSV according to SEQ ID NO: 2 (shown in lowercase letters). The respective loop modification regions are highlighted. (B) Structural alignment of the crystal structures of the capsid protein of NωV (PDB ID 7ANM, chain A; shown in green) and the capsid protein of HaSV (PDB ID 3S6P, chain A; shown in red) obtained using the RCSB PDB Pairwise Structure Alignment tool (https://www.rcsb.org/alignment) and displayed in different orientations.

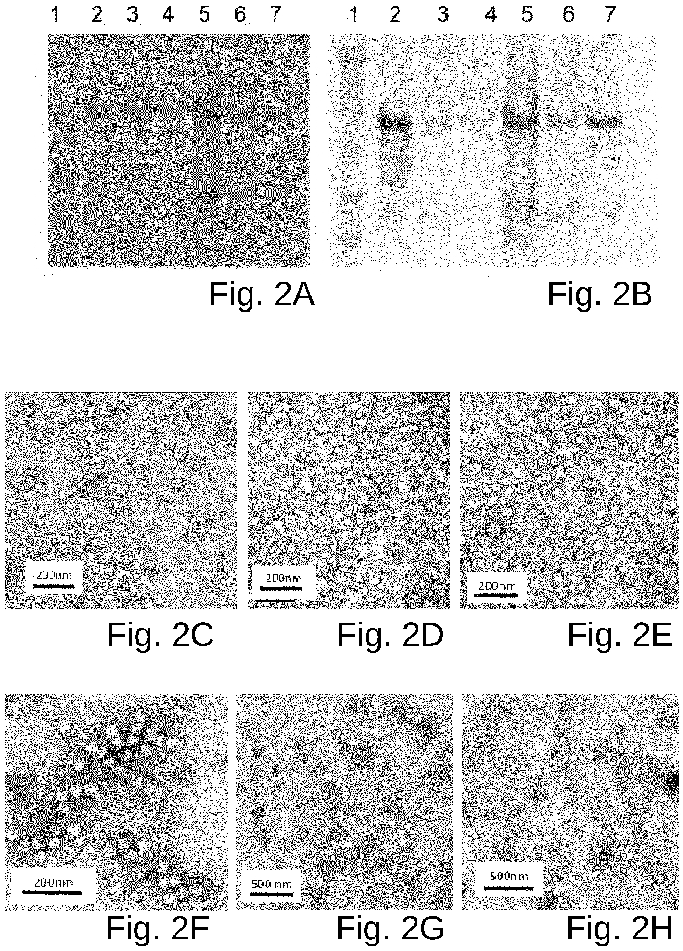

**Figure 2.** (A) Coomassie gel of constructs CP1 (lane 2), CP1-G292 Cys_I293 (lane 3), CP1-T354_Cys_T356 (lane 4), CP1-P311_Cys_G313 (lane 5), CP1-P311_MC11_G313 (lane 6) and CP1-P311_GE11_G313 (lane 7). Lane 1 is a molecular weight marker. (B) Western blot of gel shown in A. (C) TEM image of VLNP_CP1; scale bar is 200 nm. (D) TEM image of VLNP_CP1-G292 Cys_I293; scale bar is 200 nm. (E) TEM image of VLNP_CP1-T354_Cys_T356; scale bar is 200 nm. (F) TEM image of VLNP_CP1-P311_Cys_G313; scale bar is 200 nm. (G) TEM image of VLNP_CP1-P311_MC11_G313; scale bar is 500 nm. (H) TEM image of VLNP_CP1-P311_GE11_G313;_scale bar is 500 nm. While the native VLNP and constructs showing in Fig. 2E to 2H show properly folded VLNPs the construct in Fig. 2D leads to misfolded VLPs.

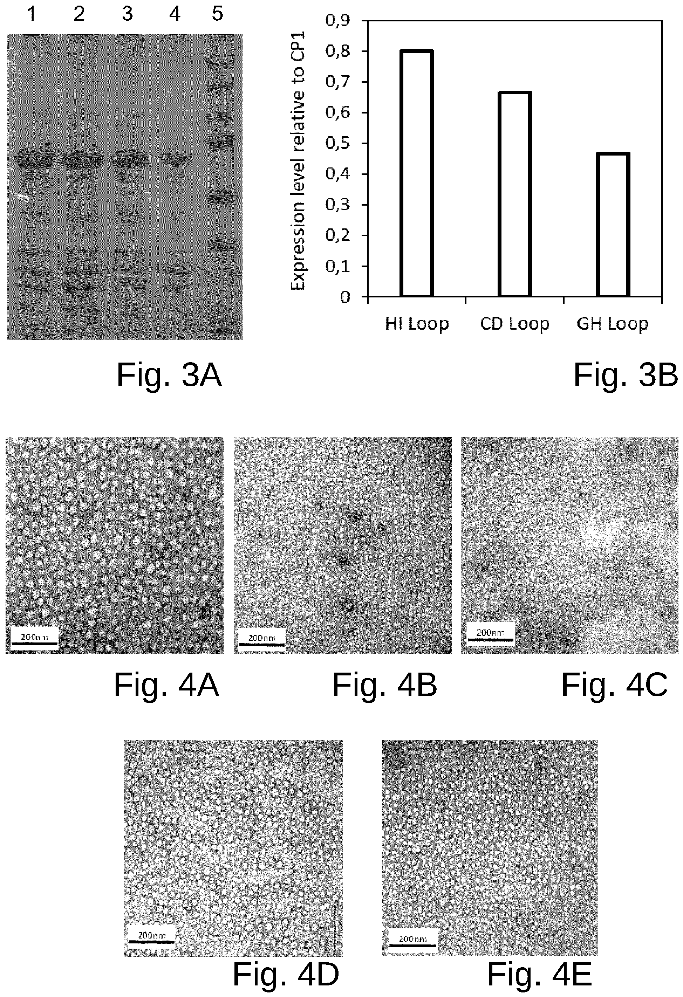

**Figure 3.** (A) Coomassie gel - lane 1: positive control CP1, lane 2: CP1-P389_GE11_T390, lane 3: CP1-P311_GE11_G313, lane 4: CP1-A378_GE11_G379, lane 5: molecular weight marker. (B) Comparison of expression yields obtained from constructs CP1-P389_GE11_T390 ("HI Loop"), CP1-P311_GE11_G313 ("CD Loop"), CP1-A378_GE11_G379 ("GH loop"); all relative to native CP1.

**Figure 4.** TEM images of constructs (A) CP1-P311_Cys_G313, (B) CP1-P389_Cys_T390, (C) CP1-A378_CPP_G379, (D) CP1-A378_MC11_G379, (E) CP1-P389 _MC11_T390; scale bar is 200 nm.

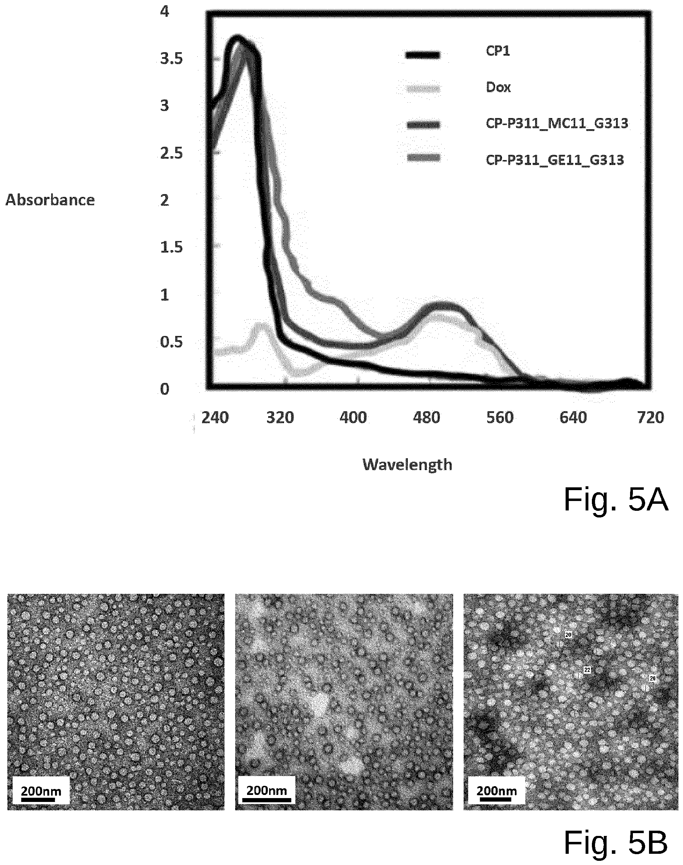

**Figure 5.** (A) UV-visible spectra of the CP1, CP1-P311_MC11_G313, CP1-P311_GE11_G313 loaded with Doxorubicin(Dox). The loaded VLNPs had a higher absorbance at 495nm as compared with unloaded VLNPs, indicating the successful loading with Dox. The entrapment efficiency (EE) of VLNP_CP1 and VLNP_CP1-P311_MC11_G31 was $5.06 \pm 0.29\%$ and $5.47 \pm 0.13\%$, respectively. The loading efficiency (LE) of VLNP-CP1 was $2.1 \pm 0.1\%$. The LE values are equivalent to $507 \pm 15$ molecules of Dox being loaded in the VLNPs. (B) TEM analysis of VLNP_CP1-Dox (left), VLNP_CP1-P311_MC11_G313-Dox (middle), and VLNP_CP1-P311_GE11_G313-Dox (right); scale bar is 200 nm.

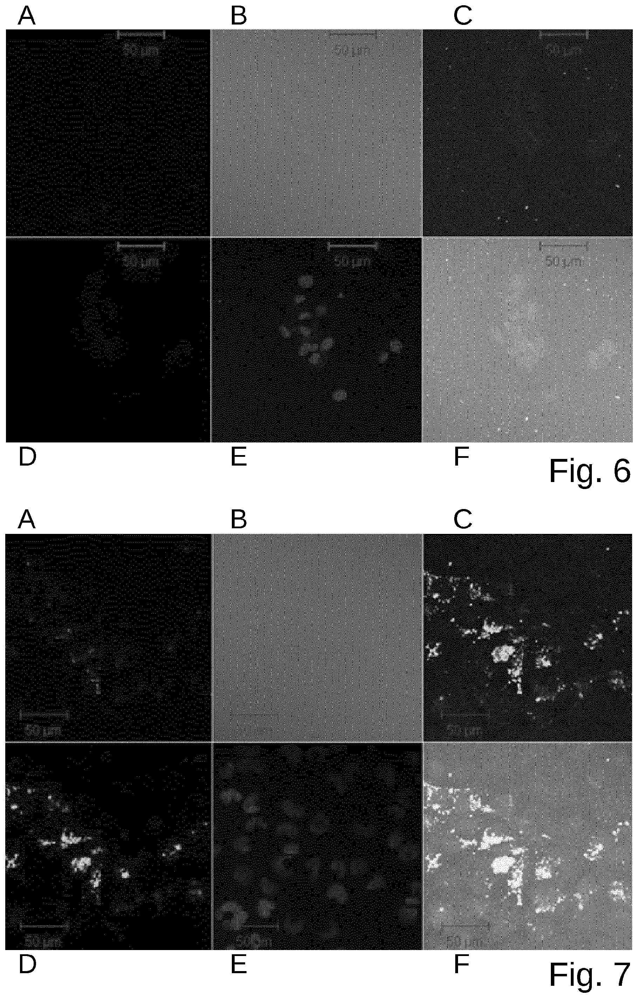

**Figure 6.** Normal cells (CCD841CoN) are unaffected by CP1-P311_Cys_G313-Dox loaded VLNP conjugated with biotin-linked MC11. (A) No staining seen for Doxorubicin. (B) Phase contrast. (C) Overlay of Dox (red) and Alexa Fluor 488 (green). (D) Alexa Fluor 488-conjugated streptavidin did not show any staining for VLNP_CP1-P311_Cys_G313 conjugated with biotin-linked MC11. (E) DAPI stained nuclei and (F) all overlay no staining in normal cells.

**Figure 7.** Bowes Melanoma Cells show targeting by CP1-P311_Cys_G313-DOX loaded VLNP conjugated with biotin linked MC11. (A) Dox fluoresecence at 550 nm, for Doxorubicin loaded CP1-P311_Cys_G313-DOX loaded VLNP conjugated with biotin linked MC11. (B) Phase contrast. (C) Overlay of Dox (red) and Alexa Fluor 647 (green) shows (yellow) co-localisation labelled overlap of Dox containing CP1-P311_Cys_G313-Dox loaded VLNP conjugated with biotin linked MC11. (D) Staining for Alexa Fluor 488-conjugated streptavidin for CP1-P311_Cys_G313-Dox loaded VLNP conjugated with biotin linked MC11. (E) DAPI stained apoptotic nuclei. (F) All overlay.

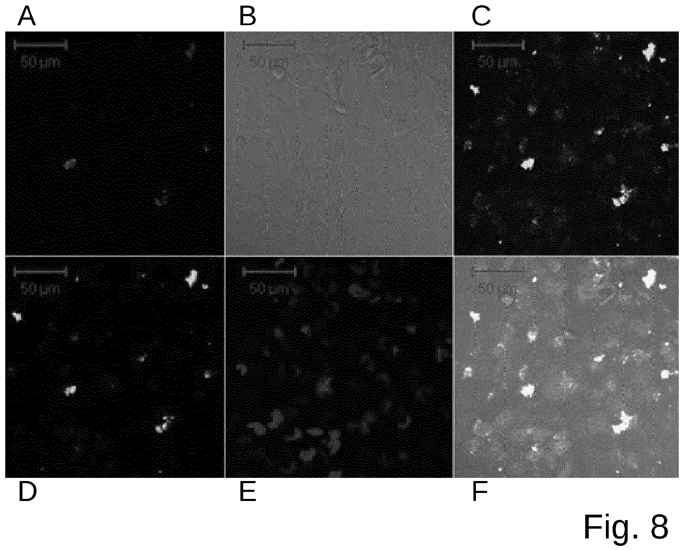

**Figure 8.** Bowes Melanoma Cells shows targeting by CP1-P311_Cys_G313-Dox loaded VLNP conjugated with biotin linked GE11. (A) Dox fluorescence of loaded VLNP conjugated with biotin linked GE11. (B) Phase contrast. (C) Overlay of DOX (red) and Alexa Fluor 647 (green) shows (yellow) co-localisation labelled overlap of Dox containing CP1-P311_Cys_G313-Dox loaded VLNP conjugated with biotin linked GE11. (D) Staining for Alexa Fluor 488-conjugated streptavidin for CP1-P311_Cys_G313-Dox loaded VLNP conjugated with biotin linked GE11. (E) DAPI stained apoptotic nuclei. (F) All overlay.

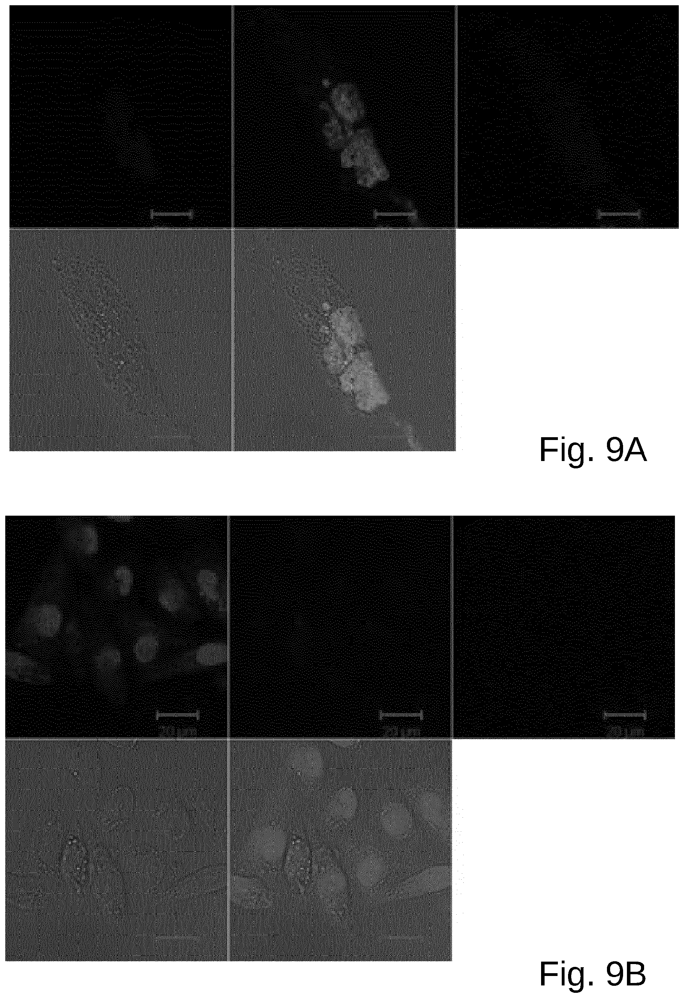

**Figure 9.** Confocal images of (A) and (B) - panels from left top to bottom right: Left top panel: Hoechst (480nm) blue fluorescence staining of nuclei; middle top panel: Dox at 550nm by its red autofluorescence; top right panel: at 630nm; bottom left panel: phase contrast; bottom right panel: overlay of all channels. The images shown are (A) for Bowes melanoma (positive control, FGF and VEGF positive) cells treated with construct Dox loaded VLNP_CP1-A378_MC11_G379 and (B) Normal cells (CCD841CoN, negative control, cells do not express FGF receptor) treated with construct Dox loaded VLNP_CP1-A378 _MC11_G379 (modified VLNP bears the FGF ligand). Bowes melanoma cells show disintegration and staining with doxorubicin, while CCD841CoN cells do not take up doxorubicin and stay viable.

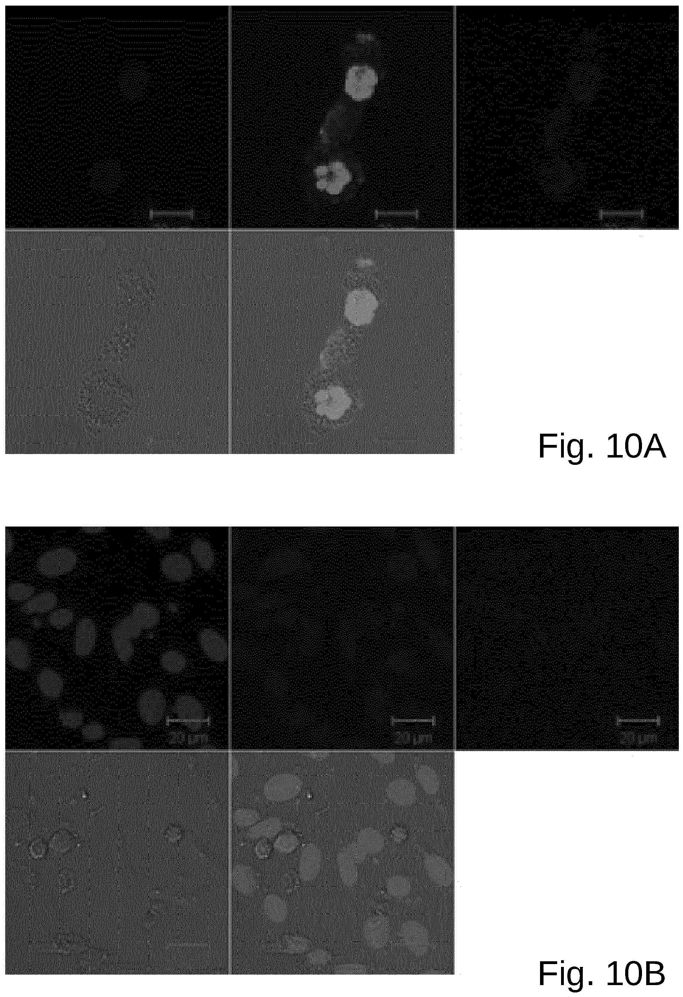

**Figure 10.** Confocal images of (A) and (B) Panels from left top to bottom right: Left top panel: Hoechst (480nm) blue fluorescence staining of nuclei; middle top panel: Dox at 550nm by its red autofluorescence; top right panel: at 630nm; bottom left panel: phase contrast; bottom right panel: overlay of all channels. The images shown are (A) for Bowes melanoma (positive control) cells treated with construct Dox loaded VLNP_CP1-A378_GE11_G379 (modified VLP bears the VEGF ligand) and (B) normal cells (CCD841CoN, negative control, cells do not express VEGF receptor) treated with construct Dox loaded VLNP _CP1-A378_ GE11_G379. Bowes melanoma cells show disintegration and staining with doxorubicin, while CCD841CoN cells do not take up doxorubicin and stay viable.

**Example 1. Materials and Methods**

For all Examples described herein, the following materials and methods were used, unless specified otherwise.

Preparation of Gene Constructs

A nucleic acid sequence encoding the 644-aa capsid protein of *Nudaurelia capensis omega virus* according to SEQ ID NO: 1 (GenBank S43937.1) was prepared as a synthetic construct, with NcoI and HindIII sites at the 5' and 3' ends, respectively, for insertion into NcoI/HindIII pET28a vector fragment (pET28a is identical to pET26b with the exception of the missing pelB signal sequence). Based on the resulting vector as a template, all further constructs (i.e., the expression vectors encoding the polypeptides according to the invention) were prepared using standard molecular biology techniques. The integrity of the recombinant vectors was confirmed by automated DNA sequencing before the transformation of *E. coli* BL21 DE3 star cells.

Expression, Purification and Assembly of VLNPs and targeting-VLNPs

The expression and purification of the polypeptides were carried out as follows. Briefly, a single colony carrying the respective expression vector was inoculated into 50 mL of Lyso-geny broth (LB) containing 50ug/mL kanamycin and was incubated for 20 h at 37 °C. A 5 mL of the preculture was used to inoculate 500 mL of LB with kanamycin. After 4-5 h of growth (OD600 nm = 0.6), expression was induced with 0.5 mM isopropyl-β-D-1-thiogalactopyranoside (IPTG), and the cells were incubated at 30 °C overnight.

The culture was centrifuged (6000g using a JLA 16.500 rotor, Beckman Coulter Inc., 10 min, 4 °C), and the pellet was suspended in 50 mM Tris buffer, pH 9. The suspension was then soni-cated and centrifuged at 36,000g using a JLA 16.200 rotor (Beck-man Coulter Inc.) at 4 °C for 1hour.

The pellet ("unwashed IBs") was resuspended in 50mM Tris, pH 9 containing 2M urea. Washing was done at 25°C in an orbital shaker at 120rpm for 1h. The IBs were re-centrifuged with 36,000g, 1h, 25°C. 1g of washed IBs were added to 5ml of 4M urea, 50mM Tris, 50mM DTT, pH9 and incubated 18 - 20 hours in an orbital shaker at 25°C and a final centrifugation step (4000g, 10min). Refolding was done in 50mM Tris, 250mM NaCl, 50mM DTT, pH 7,5 at 4 °C 48hours. Assembly was done in the presence of 75ug protein to 7.5ug capsid protein. Assembly maturation was done overnight in 200mM NaCl/200mM NaAc during maturation.

SDS-PAGE and Western blot analysis

For SDS-PAGE analysis, samples were mixed with $4\times$ loading buffer containing DTT and heated in a boiling water bath for 10 minutes prior to loading on 8% Polyacrylamide gels, electrophoresis was carried out for 1h30min, whereafter the gels were stained in Coomassie Brilliant Blue R-250 and destained. For Western blot analyses, 8% acrylamide gel and denatured proteins were electrotransferred onto Immobilon PVDF membranes. Membranes were incubated with rabbit polyclonal anti-VNLP-antibodies at a 1 : 1000 dilution. Proteins were detected by chemiluminescence after incubation with secondary goat anti-rabbit antibodies conjugated to horseradish peroxidase at a 1 : 12 500 dilution (Bio-Rad, ECL chemiluminescence kit) . Images were taken with a G : Box imaging system (Syngene, Cambridge, UK), analysed with GeneTools (Syngene) and finally processed with GIMP.

Transmission Electron Microscopy (TEM)

VLNPs were suspended at 1 mg/mL in 20 μL of potassium phosphate (KP) buffer and deposited onto Formvar carbon film coated copper TEM grids (Electron Microscopy Sciences) for 2 min at room temperature. The grids were then washed twice with deionized water for 45 secs and stained twice with 2% (w/v) uranyl acetate (UAc) in deionized water for another 30 sec. A Tecnai F30 transmission electron microscope was used to analyze the samples at 300 kV.

Loading of doxorubicin (Dox) into VLNPs

Dox was loaded into VLNPs using the infusion method as described by Yildiz et al. (Journal of Controlled Release 172.2 (2013): 568-578.

Wild-type VLNPs (i.e., based on wild-type capsid protein of *Nudaurelia capensis omega virus* according to SEQ ID NO: 1) and modified VLNPs according to the invention (each 7 mg/mL) were incubated separately with Dox (0.5 mg/mL) in Tris buffer for 3 h at 25 °C, and then for 16 h at 4 °C. The mixtures (1 mL each) were dialysed in Tris Buffer (1 L, two times) at 4 °C to remove excess Dox.

Dialysed samples of Dox-loaded VLNPs were purified from the mixtures using sucrose density gradient ultracentrifugation. The dialysed mixture (1mL) was layered on top of a sucrose density gradient [8-50% (w/v)] and centrifuged at 30,000 rpm in a Beckman 50.2 Ti rotor for 3 h at 4°C. Sucrose gradients were made with a Biocomp Gradient Master and centrifuged in a Beckman SW41 rotor at 40,000 rpm for 1.25 h at 11°C. After centrifugation, sucrose gradients were fractionated on an ISCO fractionator with constant OD254 reading, a pump setting of 0.75 mL/min, and collections at 30-sec intervals. The sucrose gradient was fractionated and the concentration of VLNPs in each fraction was determined using the Bradford assay. The fractions that contained VLNPs were pooled, dialysed in Tris buffer at 4°C, and concentrated with the Vivaspin Turbo 15 (10kDa cut off, polyethersulfone membrane; Sartorius, Germany). UV-visible measurement of VLNPs was performed using a spectrophotometer (Jenway 7315, Cole-Parmer, U.K.). Absorbance from wavelength 240 to 700nm was measured. A254 were used to calculate the entrapment efficiency (EE), loading efficiency (LE), and the number of Dox per VLNP ($N_{Dox}$) using the following equations:

- EE% = weight doxorubicin loaded/weight total doxorubicin used x 100%

$$LE\% = \text{Weight doxorubicin loaded/Weight VLNPs} \times 100\%$$

- N = LE x (molecular weight VLNP / molecular weight doxorubicin)

Dox efflux:

To examine drug efflux, Dox-loaded VLNPs were incubated in an external concentration of 200mg/ml Dox at 40°C for up to 72 hr in buffered saline gelatin (BSG) before drug extraction and quantitation, to ascertain if any drug efflux occurred. Drug extraction from packaged VLNPs involved five cycles of vortexing and sonication in the presence of 97mM HCl-isopropyl alcohol (HCl-IPA). The samples were diluted in an equal volume of water and the five cycles were repeated. After centrifugation at 13,200 rpm for 5min to pellet debris, the supernatants were harvested for drug quantitation. Drugs extracted from VLNPs were quantitated based on HPLC-fluorescence peak and LC-MS/MS analyses as previously described for Dox (Zheng et al. "Time-and concentration-dependent penetration of doxorubicin in prostate tumors." AAPS Pharmsci 3.2 (2001): 69-77). The number of molecules of Dox packaged per VLNP was calculated before targeting experiments, to standardize the amount of drug and targeting modules for each experiment.

Bioconjugation Reactions Chemical conjugation of maleimide-linked MC11 and maleimide-linked GE11 peptides onto VLNPs via thiol reaction was carried out as follows: Maleimide-linked biotinylated peptides (20-200 μM) were used to react with VLNPs (4-40 μM) in 0.01 M PBS, pH = 7.2 at 4°C overnight. Unbound maleimide-biotin-peptide was then removed using a 7000 MWCO desalting column (Ze-baTM Spin Desalting Columns, Thermo Scientific, MA, USA). As biotinylated peptides biotin-Ahx-MC11-Ahx-maleimide-Cys and biotin-Ahx-MC11-Ahx-maleimide-Cys were used. As fluorescently labelled peptides FITC-Ahx-MC11-Ahx-maleimide-Cys and FITC-Ahx-MC11-Ahx-maleimide-Cys were used.

Confocal Microscopy

For confocal fluorescence microscopy, a Zeiss LSM Meta 510 NLO, confocal microscope was used to view the treated cells. The confocal images of both the tumor and normal cells were all standardized to the same settings and images were taken at these settings. Doxorubicin loaded cells were visualised by the red autofluorescence at 561 nm laser and rhodamine filter, and the 2-photon laser at 750nm for Alexafluor 488 and the bisbenzimid-azole dye, Hoechst 33342/DAPI at 480nm with a DAPI filter was used to identify normal versus apoptotic nuclei. Targeting of VLNPs to cells were visualised with Alexa Fluor 488-conjugated streptavidin.

Confocal images were processed using ImageJ v1.44o.

Nomenclature of capsid proteins and VLNPs

In the Examples described herein, a consistent nomenclature is used for referring to different capsid protein (CP) constructs and VLNPs. Constructs based on the capsid protein sequence according to SEQ ID NO: 1 are generally referred to as "CP1", whereas constructs based on the capsid protein sequence according to SEQ ID NO: 2 are referred to as "CP2". The nomenclature is explained in the following examples:
Wild-type capsid protein of the sequence according to SEQ ID NO: 1 is simply referred to as "CP1". VLNPs assembled from wild-type CP1 are referred to as "VLNP_CP1".

To be able to dock specific compounds to the loop modification region defined by residues T374 to A382 ("GH-loop") of the capsid protein sequence according to SEQ ID NO: 1, T380 may be mutated to a cysteine using site directed mutagenesis by standard molecular biology techniques. The resulting capsid protein construct is referred to as "CP1-G379_Cys_N381" (based on the residues of SEQ ID NO: 1 flanking the inserted Cys-residue); VLNPs assembled from this construct are referred to as "VLNP_CP1-G379_Cys_N381". When the cysteine is conjugated to GE11-biotin through maleimide chemistry, the resulting VLNP is referred to as "VLNP _CP1-G379_Cys_N381 [GE11_biotin]".

In a similar way, a peptide such as the MC11-peptide (MQLPLATGGGC) may be inserted in the loop modification region defined by residues A310 to I318 (CD loop), for instance, by insertion between residues P311 and P315, replacing residues S312-D314. Such a construct is herein referred to as "CP1-P311_MC11_P315" (based on the residues of SEQ ID NO: 1 flanking the inserted MC11-peptide); the VLNPs assembled from these constructs are referred to as "VLNP _CP1-P311_MC11_P315".

A VLNP, in which both a cysteine residue is inserted in place of T380 and the MC11-peptide is inserted between residues P311 and P315, would accordingly be termed "VLNP_CP1-P311–MC11_P315-G379_Cys_N381", etc.

**Example 2. Expression, Purification and Assembly of VLNPs**

Expression and purification of CPs

The following capsid proteins (CPs) were constructed, expressed and purified by refolding from inclusion bodies, as described in Example 1:

-    wild-type CP1 ("CP1");
-    CP1-G292_Cys_I293 (corresponding to AB loop);
-    CP1-T354_Cys_T356 (corresponding to F'F'' loop);
-    CP1-P311_Cys_G313 (corresponding to CD loop);
-    CP1-P311_MC11_G313 (corresponding to CD loop);
-    CP1-P311_GE11_G313 (corresponding to CD loop).

The constructs CP1-T354_Cys_T356, CP1-P311_Cys_G313, CP1-P311_MC11_G313, and CP1-P311_GE11_G313 all contain peptide insert sequences or cysteine residues in loop modification regions as defined in the context of the present invention, more specifically, in the AB, the F'F'', and the CD loop. Construct CP1-G292_Cys_I293, on the other hand, contains a cysteine residue in the AB loop as defined in Munshi et al. ("The 2.8 Å structure of a T= 4 animal virus and its implications for membrane transloca-tion of RNA." J. Mol. Biol 261.1 (1996): 1-10), which loop is not a loop modification region as defined herein but part of a core region.

The yield of the produced CPs was evaluated by SDS-PAGE and Western blotting. The results are displayed in Fig. 2A and 2B. As can be seen from these results, all the constructs, CP1-G292_Cys_I293, CP1-T354_Cys_T356, CP1-P311_Cys_G313, CP1-P311_MC11_G313, CP1-P311_GE11_G313 showed the correct maturation size of ±64kDa in comparison to the mature native CP1. The yield obtained with CP1-G292 Cys_I293 (AB loop) was low. By contrast, the CD loop constructs CP1-P311_Cys_G313, CP1-P311_MC11_G313 and CP1-P311_GE11_G313 showed very high yields.

Assembly of VLNPs

Next, VLNPs were assembled, essentially as described in Example 1. The VLNPs were analysed by TEM, as shown in Fig. 2C-2H. As can be seen from the figures; CP1-P311_Cys_G313, CP1-P311_MC11_G313, CP1-P311_GE11_G313 formed the correct size capsids of ± 45nm when compared to the native CP1 (Fig. 2E to Fig, 2H). By contrast, VLNP_CP1-G292 Cys_I293, which contains a cysteine residue in a peptide loop belonging to a core region rather than a loop modification region as defined herein, does not show proper folding (Fig. 2D).

**Example 3. Comparison of Expression Yields obtained with different VNLP constructs**

Comparison of Expression Yields by SDS PAGE

In order to compare the expression yields obtained with modifying different loop modification regions, the following capsid proteins (CPs) were constructed, expressed and purified by refolding from inclusion bodies, essentially as described in Example 1:

- wild-type CP1 ("CP1");
- CP1-P389_GE11_T390 (corresponding to HI loop);
- CP1-P311_GE11_G313 (corresponding to CD loop);
- CP1-A378_GE11_G379 (corresponding to GH loop).

The yield of the produced CPs was evaluated by SDS-PAGE and Western blotting. The results are displayed in Fig. 3A. As can be seen from these results, among the loop modified variants, the modification in the HI loop resulted in the highest yield, followed by the CD loop and the GH loop.

Quantification of Expression Yields by Bradford Analysis

In order to compare the expression yields obtained with different CPs more quantitatively, a further experiment was conducted with the four CPs mentioned above. All CPs were expressed and purified by refolding from inclusion bodies under the same conditions. Next, a Bradford assay was done to determine the concentration of the CPs, using bovine serum albumin (BSA) as a standard. The following results were obtained (see also Fig. 3B) :

| Construct | Concentration | Relative to CP1 |
|---|---|---|
| wild-type CP1 | 0.1508 mg/mL | 1 |
| CP1-P389 GE11 T390 (HI loop) | 0.1245 mg/mL | 0,80 |
| CP1-P311 GE11 G313 (CD loop) | 0.1036 mg/mL | 0,67 |
| CP1-A378 GE11 G379 (GH loop) | 0.0714 mg/mL | 0,47 |

As can be seen from the above table, while surprisingly high yields were obtained for all three constructs, there were still striking differences between them. The highest yield was obtained with the modified HI loop (80 % of wild-type), followed by the modified CD loop (67 %) and the modified GH loop (47%).

**Example 4. TEM analysis of further VNLP constructs**

In order to test the assembly of even more VNLPs, the following capsid proteins (CPs) were constructed, expressed and purified by refolding from inclusion bodies, as described in Example 1:

- CP1-P311_Cys_G313 (corresponding to CD loop);
- CP1-P389_Cys_T390 (corresponding to HI loop);
- CP1-A378_CPP_G379 (corresponding to GH loop);
- CP1-A378_MC11_G379 (corresponding to GH loop); and
- CP1-P389_MC11_T390 (corresponding to HI loop).

wherein CPP is arginine-rich peptide of Flock House Virus (FHV), which is known to function as a cell penetrating peptide (CPP), and has the amino acid sequence: RRRRNRTRRNRRRVR (SEQ ID NO: 3).

Next, VLNPs were assembled, essentially as described in Example 1. The VLNPs were analysed by TEM, also as described in Example 1. The resulting assembled VLNPs are shown in Fig. 4A-4E.

**Example 5. Loading of VLNPs with Doxorubicin**

Loading of VLNPs with Dox was carried out as described in Example 1.

Fig. 5A shows UV-vis spectra of wild-type VLNP_CP1 as well as of the constructs VLNP_CP1-P311_MC11_G313 and VLNP_CP1-P311_GE11_G313 with Dox loaded (Construct CP-P311_MC11_G313 and CP-P311-GE11_G313) and CP1 without loaded Dox. The loaded VLNPs had a higher absorbance at 495nm as compared with unloaded VLNPs, indicating the successful loading with Dox. The entrapment efficiency (EE) of VLNP_CP1-P311_MC11_G31 was 5.06±0.29% and 5.47±0.13% for VLNP_CP1-P311_GE11_G313, respectively. The loading efficiency (LE) of VLNP_CP1-P311_MC11_G313 and VLNP_CP1-P311_GE11_G313was 2.1±0.1%. The LE values are equivalent to 507±15 molecules of Dox being loaded in the VLNPs.

The loaded VLNPs were again analysed by TEM, as shown in Fig. 5B. The analysis revealed that the VLNPs remained intact also after being loaded with Dox.

## Example 6. Targeted Doxorubicin Delivery to Tumor Cells

Tissue Culture

To determine if Dox drug-packaged VLNPs can deliver bioactive drug intracellularly into human cancer cells, we incubated various human cancer cell lines as well as normal CCD841CoN and CHO (Chinese hamster ovary) cell lines with the various Dox-loaded VLNPs as well as control VLNPs and monitored their fate over 48 hr by confocal microscopy. As controls, Dox-loaded wild-type VLNPs (based on wild-type capsid protein of *Nudaurelia capensis omega virus* according to SEQ ID NO: 1 (i.e., VLNP_CP1 loaded with Dox) without any targeting motif and non-Dox loaded VLNPs were used.

The doxorubicin loaded VLNPs and control VLNPs were diluted in an appropriate amount of culture medium before incubation overnight with the cancer cell lines and the normal control cell lines. The human 1542T prostate-, BM1604 prostate-, Bowes melanoma, MeWo melanoma, RT4 bladder-, UCT-B1 breast, Rogina breast-, A549 lung, HeLa cervical cancer cell lines were used. The normal cell lines used in this study included the CHO cell line as well as normal human CCD841CoN cell lines. All the tumor cell lines were positive for EGFR and FGFR overexpression, which were used as targets for ligand targeting of the respective VLNP constructs. Cell lines were incubated in the appropriate media with the specified amount of FBS and 100 U/ml of both penicillin G and streptomycin.

To determine the localization of VLNPs, primary antibodies specific for the coat protein together with a secondary anti-rabbit AlexaFluor 488 were used. In parallel, streptavidin tagged AlexaFluor 488 antibodies were used for the for the peptide-conjugated VLNP constructs containing biotin, whereby we were able to evaluate the exact localization of the VLNPs.

Analysis of Targeted VLNPs:

Bowes Melanoma and CCD841CoN cell lines were grown on glass coverslips in 6-well plates. The cells were incubated with media (1mL) containing non-Dox loaded VLNP_CP1-P311_Cys_G313-GC11_biotin or VLNP_CP1-P311_Cys_G313-MC11_biotin, or Dox-loaded VLNP-P311_Cys_G313-GC11_biotin or VLNP_CP1-P311_Cys_G313-MC11_biotin with an equivalent concentration (5pg/mL) of Dox for 1h, followed by washing with PBS. The cells were then fixed with paraformaldehyde [3.7% (w/v) in PBS] for 10min at 25°C, and washed with PBS. The nuclei were stained with Hoechst 33342 (2 drops/mL in PBS; Life Technologies, U.S.A.) for 10min at 25°C. After washing with PBS, the coverslips were mounted on glass slides and viewed under a confocal fluorescence microscope (Zeiss, LSM) .

Analysis of Peptide conjugated VLNPs:

Cells (25,000 cells/well) were grown for 24 hours on glass coverslips placed in an untreated 24-well plate in 200 μL media at 37°C, 5% CO2. The cells were washed twice with Dulbecco's PBS (DPBS) before addition of the VLNPs with Dox or without Dox as controls (2.5 × 10^6 particles per cell, corresponding to -0.5 μg/well) and incubation for 6-12h. The cells were washed twice in DPBS to remove unbound particles and fixed for 5 min at room temperature in DPBS containing 4% (v/v) paraformaldehyde and 0.3% (v/v) glutaraldehyde. To determine the localization of VLNPs; primary antibodies specific for the coat protein together with a secondary anti-rabbit AlexaFluor 488 were used. In parallel, streptavidin tagged AlexaFluor 488 antibodies were used for the for the peptide-conjugated VLNP constructs containing biotin, whereby we were able to evaluate the exact localization of the VLNPs. Finally, the cells were washed three times with DPBS, and the coverslips were mounted on glass slides using Fluoro-shield with 4',6-diamidino-2-phenylindole (DAPI, Sigma-Aldrich) and sealed using nail polish.

Cytotoxicity of doxorubicin (Dox) loaded inside VLNPs

Cytotoxicity of ATV-VLNPs particles with DOX or without DOX as controls was evaluated using the MTT cell viability assay. HT29 (2.0x104 cell/well), HepG2 (2.0x104 cell/well), and CCD841CoN (3.0×104 cell/well) cells were seeded in 96-well plates. After 24h, the culture media were aspirated, and the cells were added with media (100pL) containing VLNPs particles with Dox or without Dox at 10-fold serial dilutions (0.001-100μM), and incubated for 3h. Then, the solution in each well was replaced with fresh media without Dox (100pL), and the cells were incubated for 72h. After that, MTT reagent (5mg/mL in PBS; 20 μL) was added to the wells and incubated for 3h. Dimethyl sulfoxide (DMSO; 100pL) was then added to the wells to dissolve the formazan crystal formed by viable cells. Absorbance was measured using a microtiter plate reader (ELx800, Bio-Tek Instruments, U.S.A.).

Cell viability and mechanism of cell death

In order to measure the ability of the VLNPs loaded with Dox to cause cell death, a cell viability/necrosis/apoptosis assay (ApoTox-GloTM Triplex Assay, Promega, catalog nr. TM322) was conducted in normal human CCD841CoNcell line and Bowes melanoma tumor cell line. Confluent monolayers in 96-well (black) plates were infected at 8, 24 and 48 hours post seeding (100 TCID50) and cellular markers for cell viability, apoptosis and necrosis measured at 72 hpi, according to the manufacturers' instructions, using a luminometer (i.e. bioluminescence and/or fluorescence; Glomax, Promega).

The results taken together show that appropriate modification of the native VLNP CP1 and CP2 would lead to correct maturation and folding with tight encapsulation of active ingredients. The high specificity allows the targeted killing of tumor cells while non targeted cells stay completely viable. These data clearly indicate the potential for treatment of cancer using modified VLNP.

**Claims**

1.  A polypeptide comprising a capsid protein sequence consisting of eight core regions and seven loop modification regions,

    wherein the eight core regions are defined, respectively, by amino acid positions M1 to S309, P319 to R328, T340 to T342, M349 to F352, W359 to F362, V368 to Q373, T383 to T387 and T392 to N644 in the amino acid sequence according to SEQ ID NO: 1,
    wherein the eight core regions of the capsid protein sequence have at least 70 % sequence identity to the eight core regions of the amino acid sequence according to SEQ ID NO: 1 or SEQ ID NO: 2 at least in the range from R32 to N570,
    wherein the seven loop modification regions are defined, respectively, by amino acid positions A310 to I318, H329 to I339, I343 to T348, D353 to E358, A363 to D367, T374 to A382, and A388 to V391 in the amino acid sequence according to SEQ ID NO: 1,
    wherein at least one loop modification region of the capsid protein sequence differs from the corresponding loop modification region of the amino acid sequences according to SEQ ID NO: 1 and SEQ ID NO: 2 at least in that the at least one loop modification region comprises

    - a peptide insert sequence comprising at least 3 amino acids, so that the at least one loop modification region of the capsid protein sequence is extended with respect to the corresponding loop modification region of the amino acid sequences according to SEQ ID NO: 1 and SEQ ID NO: 2 by said number of amino acids; and/or
    - a cysteine residue,

    wherein the amino acid numbering corresponds to the amino acid sequence according to SEQ ID NO: 1.

2.  The polypeptide according to claim 1, wherein the at least one loop modification region of the capsid protein sequence that differs from the corresponding loop modification region of the amino acids sequences according to SEQ ID NO: 1 and SEQ ID NO: 2 is the loop modification region defined by residues A388 to V391 in the amino acid sequence according to SEQ ID NO: 1 and SEQ ID NO: 2, wherein the amino acid numbering corresponds to the amino acid sequence according to SEQ ID NO: 1.

3.  The polypeptide according to claim 1, wherein the at least one loop modification region of the capsid protein sequence that differs from the corresponding loop modification region of the amino acids sequences according to SEQ ID NO: 1 and SEQ ID NO: 2 is the loop modification region defined by residues A310 to I318 in the amino acid sequence according to SEQ ID NO: 1 and SEQ ID NO: 2, wherein the amino acid numbering corresponds to the amino acid sequence according to SEQ ID NO: 1.

4.  The polypeptide according to any one of claims 1 to 3, wherein at least two, preferably at least three, more preferably at least four, most preferably at least five of the seven loop modification regions of the capsid protein sequence differ from the corresponding loop modification region of the amino acid sequences according to SEQ ID NO: 1 and SEQ ID NO: 2 at least in that each of said loop modification regions comprises

- a peptide insert sequence comprising at least 3 amino acids, so that the loop modification region is extended with respect to the corresponding loop modification region of the amino acid sequences according to SEQ ID NO: 1 and SEQ ID NO: 2 by said number of amino acids; and/or
- a cysteine residue.

5. The polypeptide according to any one of claims 1 to 4, wherein the/each peptide insert sequence and/or the/each chemical compound is a receptor ligand, preferably selected from MC11, GE11 peptide and/or somatostatin.

6. A nucleic acid molecule comprising a sequence encoding the polypeptide according to any one of the preceding claims.

7. An expression vector comprising the nucleic acid molecule according to claim 6.

8. A host cell comprising the expression vector according to claim 7.

9. A method for producing the polypeptide according to any one of claims 1 to 5 comprising culturing the host cell according to claim 8 and recovering the polypeptide.

10. A Virus-like Nanoparticle (VLNP) comprising the polypeptide according to any one of claims 1 to 5.

11. The VLNP according to claim 10, wherein the VLNP comprises at least a first polypeptide according to any one of claims 1 to 5, and at least a second polypeptide according to any one of claims 1 to 5, wherein the first polypeptide differs from the second polypeptide.

12. The VLNP according to claim 10 or 11, wherein the VLNP comprises a payload, preferably wherein the payload is an active agent, especially a cytotoxic agent, an anti-infectious agent, and/or an anti-inflammatory agent; a detectable marker, preferably a radionucleotide; a peptide; a protein; and/or a natural or synthetic nucleic acid, in particular selected from the group consisting of nucleic acids encoding a desired protein such as mRNA, cDNA, a plasmid or vector, inhibitory nucleic acids such as siRNA or miRNA and/or nucleic acids having catalytic activity such as a ribozyme.

13. A method for producing a VLNP according to any one of claims 10 to 12, comprising the steps of

- providing the polypeptide according to any one of claims 1 to 5 in the form of inclusion bodies (IBs);
- solubilizing said IBs;
- refolding the polypeptide; and
- allowing the VLNP to spontaneously assemble from the polypeptide,

preferably wherein the VLNP is allowed to spontaneously assemble from the polypeptide in the presence of a payload.

14. A composition comprising the VLNP according to any one of claims 10 to 12 and a pharmaceutically acceptable excipient.

15. The composition according to claim 14 for use in medicine, preferably for use as a vaccine and/or in the treatment and/or in the diagnosis of cancer, especially wherein the composition is used as standalone therapy or in combination with other suitable therapeutics.

```
  1 -----MDSNSASGKRRSRNVRIAANTVNVAPKQRQARGRRARSRANNIDN    45
       :..|......:|.:||||::||||.|..::.|.  ||...:..||
  1 mgdagvasqrphnrrgtrnvrvsantvtvngrrnqr--rrtgrqvsppdn    48

 46 VTAAAQELGQSLDANVITFPTNVATMPEFRSWARGKLDIDQDSIGWYFKY    95
     .|||||:|.||||||.:|||.|:::|||||:||:||:|:|.||||||||||
 49 ftaaaqdlaqsldantvtfpanissmpefrnwakgkidldsdsigwyfky    98

 96 LDPAGATESARAVGEYSKIPDGLVKFSVDAEIREIYNEECPTVSDASIPL   145
     |||||||||||||||||||||||||||||||||||||||||.|:|.|:||
 99 ldpagatesaravgeyskipdglvkfsvdaeireiyneecpvvtdvsvpl   148

146 DGAQWSLSIISYPMFRTAYFAVANVDNKEISLDVTNDLIVWLNNLASWRD   195
     ||.|||||||.|:|||||||.|||||:|||:||||.||||.||||||.||.
149 dgrqwslsifsfpmfrtayvavanvenkemsldvvndliewlnnladwry   198

196 VVDSGQWFTFSDDPTWFVRIRVLHPTYDLPDPTEGLLRTCSDYRLTYKSI   245
     ||||.||..|::|.|::|||||||.||||:|||||||:||.||||||||:|
199 vvdseqwinftndttyyvrirvlrptydvpdpteglvrtvsdyrltykai   248

246 TCEANMPTLVDQGFWIGGHYALTPIATTQNAVEGSGFEHPFNVTRPGIAA   295
     ||||||||||||||||||.|||||.:..|..|..:...|.....||..||
249 tceanmptlvdqgfwiggqyaltptslpqydvseayalhtltfarpssaa   298

296 GVTLTWASMPPGGSAPSGDPAWIPDSTTQF-QWRHGGFDAPTGVITYTIP   344
     .:...||.:|.||:||:|.|||...|:..: .|||.|....|.|.::|.:|
299 alafvwaglpqggtapagtpaweqassggyltwrhngttfpagsvsyvlp   348

345 RGYTMQYFDTTTNEWNGFANPDDVVTFGQ--------TGGAAGTNATITI   386
     .|:..::.:|.....|..||:..|.|||.|        |...||..
349 egfalerydpndgswtdfasagdtvtfrqvavdevvvtnnpaggg-----   393

387 TAPTVTLTILATTTSAANVINFRNLDAETTAASNRSEVPLPPLTFGQTAP   436
     :|||.|:..: ..:.:..|.:  |||...||..:|.|.|.|:|:||..||||..
394 saptftvrv-ppsnaytntv-frntlletrpssrrlelpmppadfgqtva   441

437 NNPKIEQTLVKDTLGSYLVHSKMRNPVFQLTPASSFGAISFTNPGFDRNL   486
     |||||||:|:|:|||.||||||||||||||||||||||||:||.|||::|..
442 nnpkieqsllketlgcylvhskmrnpvfqltpassfgavsfnnpgyertr   491

487 DLPGFGGIRDSLDVNMSTAVCHFRSLSKSCSIVTKTYQGWEGVTNVNTPF   536
     |||.:.|||||.|.||||||.||||||.|||||||||||||||||||||||
492 dlpdytgirdsfdqnmstavahfrslshscsivtktyqgwegvtnvntpf   541

537 GQFAHSGLLKNDEILCLADDLATRLTGVYGATDNFAAAVLAFAANMLTSV   586
     ||||||:||||||:|||||||||||||||||||.||||||||.||||||:||
542 gqfahagllkneeilcladdlatrltgvypatdnfaaavsafaanmlssv   591

587 LKSEATTSVIKELGNQATGLANQGLARLPGLLASIPGKIAARVRARRDRR   636
     ||||||:|:||.:|..|.|.|..|||:||||.|:||||||.|:|||||||.||
592 lkseatssiiksvgetavgaaqsglaklpgllmsvpgkiaarvrarrarr   641

637 RAARMNNN      644
     ||||.|
642 raaran--      647
```

Fig. 1A

Fig. 1B

Fig. 2A

Fig. 2B

Fig. 2C

Fig. 2D

Fig. 2E

Fig. 2F

Fig. 2G

Fig. 2H

Fig. 3A

Fig. 3B

Fig. 4A          Fig. 4B          Fig. 4C

Fig. 4D                Fig. 4E

Fig. 5A

Fig. 5B

Fig. 6

Fig. 7

Fig. 8

Fig. 9A

Fig. 9B

Fig. 10A

Fig. 10B

Europäisches
Patentamt

European
Patent Office

Office européen
des brevets

# EUROPEAN SEARCH REPORT

**Application Number**

EP 24 17 4890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MAREE ET AL.: "Surface display of an internal His-tag on virus-like particles of Nudaurelia capensis w virus (NwV) produced in a baculovirus expression system", JOURNAL OF VIROLOGICAL METHODS, vol. 136, no. 1-2, 2006, pages 283-288, XP027892389, *Abstract* * page 287, column 2, paragraph 2; figures 1-5 * | 1,6-10, 12,14,15 | INV. C07K14/005 C12N7/00 |
| X | US 2007/160628 A1 (BIRKETT ASHLEY J [US] ET AL) 12 July 2007 (2007-07-12) * example 13 * | 1,5-10, 12,14,15 | |
| Y,D | WO 97/46666 A1 (COMMW SCIENT IND RES ORG [AU]; GORDON KARL HEINRICH [AU] ET AL.) 11 December 1997 (1997-12-11) *Abstract* * page 6, line 5 - page 7, line 14 * * page 16, line 14 - page 17, line 12 * * figures 1, 13, 18, 26, 27 * * examples 1-3, 5 * | 1-15 | |
| Y,D | HELGSTRAND C ET AL: "The refined structure of Nudaurelia capensis @w Virus reveals control elements for a T = 4 capsid maturation", VIROLOGY, ELSEVIER, AMSTERDAM, NL, vol. 318, no. 1, 5 January 2004 (2004-01-05), pages 192-203, XP004816877, ISSN: 0042-6822, DOI: 10.1016/J.VIROL.2003.08.045 *Abstract* * figure 2 * | 1-15 | |
| | -/-- | | |

|  | | | **TECHNICAL FIELDS SEARCHED (IPC)** |
|---|---|---|---|
| | | | C07K A61K C12N |

The present search report has been drawn up for all claims

| Place of search | Date of completion of the search | Examiner |
|---|---|---|
| Munich | 23 October 2024 | Mabit, Hélène |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

**EUROPEAN SEARCH REPORT**

Application Number

EP 24 17 4890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | MUNSHI S ET AL: "THE 2.8 A STRUCTURE OF A T=4 ANMIAL VIRUS AND ITS IMPLICATIONS FOR MEMBRANE TRANSLOCATION OF RNA", JOURNAL OF MOLECULAR BIOLOGY, ACADEMIC PRESS, UNITED KINGDOM, vol. 261, 1 January 1996 (1996-01-01), pages 1-10, XP001095823, ISSN: 0022-2836, DOI: 10.1006/JMBI.1996.0437 *Abstract* * figure 1 * | 1-15 | |
| Y | WO 2015/179321 A2 (UNIV CALIFORNIA [US]) 26 November 2015 (2015-11-26) * claims 1-46 * | 1-15 | |
| A,D | AGRAWALJOHNSON: "Sequence and analysis of the capsid protein of Nudaurelia capensis w virus, an insect virus with T= 4 icosahedral symmetry", VIROLOGY, vol. 190, no. 2, 1992, pages 806-814, XP023057101, *Abstract* * figure 3 * | 1-15 | TECHNICAL FIELDS SEARCHED (IPC) |
| A | WO 94/04660 A1 (COMMW SCIENT IND RES ORG [AU]; PACIFIC SEEDS PTY LTD [AU] ET AL.) 3 March 1994 (1994-03-03) *Abstract* * page 62, line 5 - line 27 * * page 72, line 1 - line 27 * * figure 2 * * example 6 * | 1-15 | |

-/--

The present search report has been drawn up for all claims

| Place of search | Date of completion of the search | Examiner |
|---|---|---|
| Munich | 23 October 2024 | Mabit, Hélène |

EPO FORM 1503 03.82 (P4C01)

Europäisches
Patentamt

European
Patent Office

Office européen
des brevets

# EUROPEAN SEARCH REPORT

**Application Number**

EP 24 17 4890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BERARDI ALBERTO ET AL: "High stability of plant-expressed virus-like particles of an insect virus in artificial gastric and intestinal fluids", EUROPEAN JOURNAL OF PHARMACEUTICS AND BIOPHARMACEUTICS, vol. 155, 1 October 2020 (2020-10-01), pages 103-111, XP055936753, NL ISSN: 0939-6411, DOI: 10.1016/j.ejpb.2020.08.012 *Abstract* * page 110, column 1, paragraph 2 * | 1-15 | |
| Y,D | NKANGA CHRISTIAN ISALOMBOTO ET AL: "The pharmacology of plant virus nanoparticles", VIROLOGY, ELSEVIER, AMSTERDAM, NL, vol. 556, 28 January 2021 (2021-01-28), pages 39-61, XP086504038, ISSN: 0042-6822, DOI: 10.1016/J.VIROL.2021.01.012 [retrieved on 2021-01-28] *Abstract* * page 42, column 1, paragraph 4 * * tables 1-3 * | 5,15 | TECHNICAL FIELDS SEARCHED (IPC) |

The present search report has been drawn up for all claims

| Place of search | Date of completion of the search | Examiner |
|---|---|---|
| Munich | 23 October 2024 | Mabit, Hélène |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 4890

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2024

| Patent document cited in search report | | Publication date | Patent family member(s) | | Publication date |
|---|---|---|---|---|---|
| US 2007160628 | A1 | 12-07-2007 | CN | 101309699 A | 19-11-2008 |
| | | | EP | 1931378 A2 | 18-06-2008 |
| | | | JP | 2009516641 A | 23-04-2009 |
| | | | US | 2007160628 A1 | 12-07-2007 |
| | | | WO | 2007027640 A2 | 08-03-2007 |
| WO 9746666 | A1 | 11-12-1997 | AT | E263234 T1 | 15-04-2004 |
| | | | CA | 2256696 A1 | 11-12-1997 |
| | | | DE | 69728447 T2 | 05-01-2005 |
| | | | EP | 1015560 A1 | 05-07-2000 |
| | | | JP | 3945823 B2 | 18-07-2007 |
| | | | JP | 2000511426 A | 05-09-2000 |
| | | | US | 6251654 B1 | 26-06-2001 |
| | | | WO | 9746666 A1 | 11-12-1997 |
| WO 2015179321 | A2 | 26-11-2015 | EP | 3145540 A2 | 29-03-2017 |
| | | | TW | 201546087 A | 16-12-2015 |
| | | | US | 2017107261 A1 | 20-04-2017 |
| | | | US | 2019031720 A1 | 31-01-2019 |
| | | | WO | 2015179321 A2 | 26-11-2015 |
| WO 9404660 | A1 | 03-03-1994 | AU | 678982 B2 | 19-06-1997 |
| | | | BR | 9306907 A | 08-12-1998 |
| | | | CA | 2141583 A1 | 03-03-1994 |
| | | | EP | 0786003 A1 | 30-07-1997 |
| | | | NZ | 254684 A | 29-01-1997 |
| | | | PL | 307474 A1 | 29-05-1995 |
| | | | RU | 95106626 A | 27-03-1997 |
| | | | US | 6177075 B1 | 23-01-2001 |
| | | | WO | 9404660 A1 | 03-03-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9746666 A1

**Non-patent literature cited in the description**

- **NKANGA et al.** The pharmacology of plant virus nanoparticles.. *Virology*, 2021, vol. 556, 39-61
- **AGRAWAL ; JOHNSON**. Sequence and analysis of the capsid protein of Nudaurelia capensis ω virus, an insect virus with T= 4 icosahedral symmetry.. *Virology*, 1992, vol. 190 (2), 806-814
- **MAREE et al.** Surface display of an internal His-tag on virus-like particles of Nudaurelia capensis ω virus (NωV) produced in a baculovirus expression system.. *Journal of Virological Methods*, 2006, vol. 136 (1-2), 283-288
- **MUNSHI et al.** The 2.8 Å structure of a T= 4 animal virus and its implications for membrane translocation of RNA.. *J. Mol. Biol*, 1996, vol. 261 (1), 1-10
- **HELGSTRAND et al.** The refined structure of Nudaurelia capensis ω Virus reveals control elements for a T= 4 capsid maturation.. *Virology*, 2004, vol. 318 (1), 192-203
- **HANZLIK et al.** Sequence of RNA2 of the Helicoverpa armigera stunt virus (Tetraviridae) and bacterial expression of its genes.. *Journal of General Virology*, 1995, vol. 76 (4), 799-811
- **WORM et al.** Targeting of peptide-binding receptors on cancer cells with peptide-drug conjugates.. *Peptide Science*, 2020, vol. 112 (3), e24171
- **LE JONCOUR et al.** Seek & Destroy, use of targeting peptides for cancer detection and drug delivery.. *Bioorganic & Medicinal Chemistry*, 2018, vol. 26 (10), 2797-2806
- **AYO et al.** Peptide-Based Strategies for Targeted Tumor Treatment and Imaging.. *Pharmaceutics*, 2021, vol. 13 (4), 481
- **LI et al.** FGF receptor-mediated gene delivery using ligands coupled to polyethylenimine.. *Journal of Biomaterials Applications*, 2007, vol. 22 (2), 163-180
- **XU et al.** GE11 peptide-conjugated nanoliposomes to enhance the combinational therapeutic efficacy of docetaxel and siRNA in laryngeal cancers.. *International Journal of Nanomedicine*, 2017, vol. 12, 6461
- **YILDIZ et al.** Infusion of imaging and therapeutic molecules into the plant virus-based carrier cowpea mosaic virus: cargo-loading and delivery.. *Journal of Controlled Release*, 2013, vol. 172 (2), 568-578
- **BIABANIKHANKAHDANI et al.** A simple add-and-display method for immobilisation of cancer drug on His-tagged virus-like nanoparticles for controlled drug delivery.. *Scientific Reports*, 2017, vol. 7 (1), 1-12
- **MASARAPU et al.** Physalis mottle virus-like particles as nanocarriers for imaging reagents and drugs.. *Biomacromolecules*, 2017, vol. 18 (12), 4141-4153
- **NOORAEI et al.** Virus-like particles: preparation, immunogenicity and their roles as nanovaccines and drug nanocarriers.. *Journal of nanobiotechnology*, 2021, vol. 19 (1), 1-27
- **RICE et al.** EMBOSS: the European Molecular Biology Open Software Suite. *Trends Genet.*, June 2000, vol. 16 (6), 276-7
- **YILDIZ et al.** *Journal of Controlled Release*, 2013, vol. 172 (2), 568-578
- **ZHENG et al.** Time-and concentration-dependent penetration of doxorubicin in prostate tumors.. *AAPS Pharmsci*, 2001, vol. 3 (2), 69-77
- **MUNSHI et al.** The 2.8 Å structure of a T= 4 animal virus and its implications for membrane transloca-tion of RNA. *J. Mol. Biol*, 1996, vol. 261 (1), 1-10